(19) Europäisches Patentamt
European Patent Office
Office européen des brevets

(11) **EP 3 230 474 B1**

(12) **EUROPEAN PATENT SPECIFICATION**

(45) Date of publication and mention
of the grant of the patent:
**23.04.2025 Bulletin 2025/17**

(21) Application number: **15867540.5**

(22) Date of filing: **09.12.2015**

(51) International Patent Classification (IPC):
***G16B 30/00*** *(2019.01)*

(52) Cooperative Patent Classification (CPC):
(C-Sets available)
**C12Q 1/6827; G16B 30/00** (Cont.)

(86) International application number:
**PCT/KR2015/013460**

(87) International publication number:
**WO 2016/093619 (16.06.2016 Gazette 2016/24)**

(54) **DETECTION OF TARGET NUCLEIC ACID SEQUENCES USING DIFFERENT DETECTION TEMPERATURES AND REFERENCE VALUES**

DETEKTION VON ZIELNUKLEINSÄURESEQUENZEN MITHILFE UNTERSCHIEDLICHER DETEKTIONSTEMPERATUREN UND REFERENZWERTE

DÉTECTION DE SÉQUENCES D'ACIDES NUCLÉIQUES CIBLES AU MOYEN DE DIFFÉRENTES TEMPÉRATURES DE DÉTECTION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.12.2014 US 201462089723 P**

(43) Date of publication of application:
**18.10.2017 Bulletin 2017/42**

(73) Proprietor: **Seegene, Inc.**
**Seoul 05548 (KR)**

(72) Inventors:
• **CHUN, Jong Yoon**
**Seoul 06075 (KR)**
• **LEE, Young Jo**
**Seoul 05507 (KR)**

(74) Representative: **Scholz, Volker**
**Boehmert & Boehmert**
**Anwaltspartnerschaft mbB**
**Pettenkoferstrasse 22**
**80336 München (DE)**

(56) References cited:
WO-A1-2010/104768       WO-A1-2015/147370
WO-A2-2012/048207       KR-B1- 101 032 750
US-A1- 2011 151 461       US-A1- 2012 202 203

• S. CHAKRAVORTY ET AL: "Rapid Detection of Fluoroquinolone-Resistant and Heteroresistant Mycobacterium tuberculosis by Use of Sloppy Molecular Beacons and Dual Melting-Temperature Codes in a Real-Time PCR Assay", JOURNAL OF CLINICAL MICROBIOLOGY, vol. 49, no. 3, 29 December 2010 (2010-12-29), US, pages 932 - 940, XP055225174, ISSN: 0095-1137, DOI: 10.1128/JCM.02271-10
• SANCHEZ, J. AQUILES ET AL.: "Two-temperature LATE-PCR endpoint genotyping", BIOMED CENTRAL (BMC) BIOTECHNOLOGY, vol. 6, no. 44, pages 1 - 14, XP021023580
• CHAKRAVORTY ET AL.: "Rapid detection of fluoroquinolone-resistant and heteroresistant Mycobacterium tuberculosis by use of sloppy molecular beacons and dual melting-temperature codes in a real-time PCR assay", JOURNAL OF CLINICAL MICROBIOLOGY, vol. 49, no. 3, 2011, pages 932 - 940, XP055225174, DOI: doi:10.1128/JCM.02271-10

(52) Cooperative Patent Classification (CPC): (Cont.)

C-Sets
**C12Q 1/6827, C12Q 2527/107, C12Q 2545/114,
C12Q 2547/101, C12Q 2561/113, C12Q 2563/107**

**Description**

**BACKGROUND OF THE INVENTION**

**FIELD OF THE INVENTION**

The present invention relates to detection of target nucleic acid sequences using different detection temperatures and reference values.

**DESCRIPTION OF THE RELATED ART**

For detection of target nucleic acid sequences, real-time detection methods are widely used to detect target nucleic acid sequences with monitoring target amplification in a real-time manner. The real-time detection methods generally use labeled probes or primers specifically hybridized with target nucleic acid sequences. The exemplified methods by use of hybridization between labeled probes and target nucleic acid sequences include Molecular beacon method using dual-labeled probes with hairpin structure (Tyagi et al, Nature Biotechnology v.14 MARCH 1996), HyBeacon method (French DJ et al., Mol. Cell Probes, 15(6):363-374(2001)), Hybridization probe method using two probes labeled each of donor and acceptor (Bernad et al, 147-148 Clin Chem 2000; 46) and Lux method using single-labeled oligonucleotides (U.S. Pat No 7,537,886). TaqMan method (U.S. Pat Nos 5,210,015 and 5,538,848) using dual-labeled probes and its cleavage by 5'-nuclease activity of DNA polymerase is also widely employed in the art.

The exemplified methods using labeled primers include Sunrise primer method (Nazarenko et al, 2516-2521 Nucleic Acids Research, 1997, v.25 no.12, and US Pat. No. 6,117,635), Scorpion primer method (Whitcombe et al, 804-807, Nature Biotechnology v.17 AUGUST 1999 and US Pat. No. 6,326,145) and TSG primer method (WO 2011/078441).

As alternative approaches, real-time detection methods using duplexes formed depending on the presence of target nucleic acid sequences have been proposed: Invader assay (US 5,691,142, US 6,358,691 and US 6,194,149), PTOCE (PTO cleavage AND extension) method (WO 2012/096523), PCE-SH (PTO Cleavage and Extension-Dependent Signaling Oligonucleotide Hybridization) method (WO 2013/115442), PCE-NH (PTO Cleavage and Extension-Dependent Non-Hybridization) method (PCT/KR2013/012312).

The conventional real-time detection technologies described above detect signals generated from fluorescent labels at a selected detection temperature in signal amplification process associated with or with no target amplification. When a plurality of target nucleic acid sequences using a single type of label in a single reaction tube are detected in accordance with the conventional real-time detection technologies, generated signals for target nucleic acid sequences are not differentiated from each other. Therefore, the conventional real-time detection technologies generally employ different types of labels for detecting a plurality of target nucleic acid sequences. The melting analysis using $T_m$ difference permits to detect a plurality of target nucleic acid sequences even a single type of label. However, the melting analysis has serious shortcomings in that its performance time is longer than real-time technologies and design of probes with different $T_m$ values becomes more difficult upon increasing target sequences.

Accordingly, where novel methods or approaches being not dependent on melting analysis for detecting a plurality of target nucleic acid sequences using a single type of label in a single reaction vessel and a single type of detector are developed, they enable to detect a plurality of target nucleic acid sequences with dramatically enhanced convenience, cost-effectiveness and efficiency. In addition, the combination of the novel methods with other detection methods (e.g., melting analysis) would result in detection of a plurality of target nucleic acid sequences using a single type of label in a single reaction vessel with dramatically enhanced efficiency.

Document WO 2015/147370 A1 (SEEGENE INC [KR]) 1 October 2015 (2015-10-01), discloses a method of detecting two target nucleic acid sequences by using different detection temperatures. However, the low detection temperature is such that both signal-generating means generate a signal, while the high temperature is such that only one, target specific signal is generated.

Throughout this application, various patents and publications are referenced and citations are provided in parentheses.

**SUMMARY OF THE INVENTION**

The present inventors have made intensive researches to develop novel methods for qualitatively or quantitatively detecting a target nucleic acid sequence, particularly a plurality of target nucleic acid sequences in more accurate and convenient manner. As a result, we have found that signals for target nucleic acid sequences are obtained at adjusted detection temperatures and then detection results are suitably interpreted by using reference values, thereby enabling to detect a plurality of target nucleic acid sequences, even using a single type of label in a single reaction vessel and a single type of detector with dramatically enhanced convenience, cost-effectiveness and efficiency.

Accordingly, it is an object of this disclosure to provide a method and a kit, which is not claimed, for detecting at least one target nucleic acid sequences of two target nucleic acid sequences in a sample using different detection temperatures and reference values.

It is another object of this disclosure to provide a method and a kit, which is not claimed, for SNP genotyping of a nucleic acid sequence in a sample using different detection temperatures and reference values.

It is still another object of this invention to provide a computer readable storage medium containing instructions to configure a processor to perform a method for determining the presence of at least one target nucleic acid sequences of two target nucleic acid sequences in a sample using different detection temperatures and reference values.

It is further object of this invention to provide a computer readable storage medium containing instructions to configure a processor to perform a method for SNP genotyping of a nucleic acid sequence in a sample using different detection temperatures and reference values.

It is still further object of this invention to provide a device for determining the presence of at least one target nucleic acid sequences of two target nucleic acid sequences in a sample using different detection temperatures and reference values.

It is another object of this invention to provide a device for SNP genotyping of a nucleic acid sequence in a sample using different detection temperatures and reference values.

It is still another object of this invention to provide a computer program to be stored on a computer readable storage medium to configure a processor to perform a method for determining the presence of at least one target nucleic acid sequences of two target nucleic acid sequences in a sample using different detection temperatures and reference values.

It is further object of this invention to provide a computer program to be stored on a computer readable storage medium to configure a processor to perform a method for SNP genotyping of a nucleic acid sequence in a sample using different detection temperatures and reference values.

Other objects and advantages of the present invention will become apparent from the detailed description to follow taken in conjugation with the appended claims and drawings.

## BRIEF DESCRIPTION OF THE DRAWINGS

Fig. 1A represents the detection results of a target nucleic acid sequence (genome DNA of *Chlamydia trachomatis,* CT), a target nucleic acid sequence (genome DNA of *Neisseria gonorrhoeae,* NG) and their combination at a relatively high detection temperature (72°C) and a relatively low detection temperature (60°C). The signals for CT and NG were generated by the TaqMan probe method.

Fig. 1B represents obtaining reference values based on the detection results of Fig. 1A. The ratio of the signal detected at the relatively low detection temperature to the signal detected at the relatively high detection temperature was used as reference values.

Fig. 1C schematically represents determination of the presence of a target nucleic acid sequence (genome DNA of *Neisseria gonorrhoeae,* NG) by using the reference value of CT obtained in Fig. 1B and the signals detected in Fig. 1A. The dotted lines denote a threshold value.

Fig. 1D schematically represents determination of the presence of a target nucleic acid sequence (genome DNA of *Chlamydia trachomatis,* CT) by using the reference value of NG obtained in Fig. 1B and the signals detected in Fig. 1A. The dotted line denotes a threshold value. The dotted lines denote a threshold value.

Fig. 2A represents the detection results of a target nucleic acid sequence (genome DNA of *Chlamydia trachomatis,* CT), a target nucleic acid sequence (genome DNA of *Neisseria gonorrhoeae,* NG) and their combination at a relatively high detection temperature (67.8°C) and a relatively low detection temperature (60°C). The signals for CT and NG were generated by the PTOCE method.

Fig. 2B represents obtaining reference values based on the detection results of Fig. 2A. The ratio of the signal detected at the relatively low detection temperature to the signal detected at the relatively high detection temperature was used as reference values.

Fig. 2C schematically represents determination of the presence of a target nucleic acid sequence (genome DNA of *Neisseria gonorrhoeae,* NG) by using the reference value of CT obtained- in Fig. 2B and the signals detected in Fig. 2A. The dotted lines denote a threshold value.

Fig. 2D schematically represents determination of the presence of a target nucleic acid sequence (genome DNA of *Chlamydia trachomatis,* CT) by using the reference value of NG obtained in Fig. 2B and the signals detected in Fig. 2A. The dotted line denotes a threshold value. The dotted lines denote a threshold value.

Fig. 3A represents the detection results of each SNP genotype (wild homozygote, mutant homozygote and heterozygote) at a relatively high detection temperature (64°C) and a relatively low detection temperature (60°C). The signals were generated by the PTOCE method.

Fig. 3B represents obtaining reference values based on the detection results of Fig. 3A. The ratio of the signal detected at the relatively low detection temperature to the signal detected at the relatively high detection temperature was used as reference values.

## DETAILED DESCRIPTION OF THIS INVENTION

The most prominent feature of the present invention is to detect a plurality of target nucleic acid sequences even using a single type of label and a single type of detector in a signal reaction vessel. The present invention employing different detection temperatures and reference values enables to detect a plurality of target nucleic acid sequences even with a single type of label in a single reaction vessel. Furthermore, the present invention enables to detect a plurality of target nucleic acid sequences even when each of target nucleic acid sequences generates signals at all of different detection temperatures. The elements of the present invention are selected in compliance with the feature of the present invention and fabricated into a surprising process for detect target nucleic acid sequences.

Conventional real-time PCR methods require two types of fluorescent labels or melting analysis for detection of two target nucleic acid sequences in a single reaction vessel.

The present invention permits real-time PCR protocols to detect two target nucleic acid sequences even using a single type of fluorescent label in a single reaction vessel.

The present invention employs our interesting findings that a reference value reflecting a difference between signals detected at a relatively high detection temperature and a relatively low detection temperature for one of two a target nucleic acid sequences can be used to determine the presence of the other target nucleic acid sequence. A reference value for a target nucleic acid sequence used in the present invention are a constant value to be empirically obtained by generating and detecting signals from a signal-generating means at a relatively high detection temperature and a relatively low detection temperature. The reference value may be directly used to detect target nucleic acid sequences. Alternatively, the reference value may be variously applied to equations which process signals for demonstrating the presence and absence of target nucleic acid sequences.

In particular, the present invention enables to detect a plurality of target nucleic acid sequences even when signal-generating means for detection of the plurality of target nucleic acid sequences generate signals at all of different detection temperatures.

The present invention can be embodied to various aspects as follows:

(a) Detection of at least one target nucleic acid sequence of two target nucleic acid sequences in a sample using different detection temperatures and reference values; and

(b) SNP genotyping of a nucleic acid sequence in a sample using different detection temperatures and reference values.

## I. Detection of At Least One Target Nucleic Acid Sequence in a Sample Using Different Detection Temperatures and Reference Values

In one aspect of this invention, there is provided a method for detecting at least one target nucleic acid sequences of two target nucleic acid sequences, as defined in the appended claims, comprising a first target nucleic acid sequence and a second target nucleic acid sequence in a sample using different detection temperatures and reference values, comprising:

(a) providing (i) a first reference value for the first target nucleic acid sequence which represents a relationship of change in signals provided at a relatively high detection temperature and a relatively low detection temperature by a first signal-generating means and/or (ii) a second reference value for the second target nucleic acid sequence which represents a relationship of change in signals provided at the relatively high detection temperature and the relatively low detection temperature by a second signal-generating means; wherein the first reference value is different from the second reference value;

(b) incubating the sample with the first signal-generating means and the second signal-generating means for detection of the two target nucleic acid sequences and detecting signals from the two signal-generating means at the relatively high detection temperature and the relatively low detection temperature; wherein the two signal-generating means generate signals at the relatively high detection temperature and the relatively low detection temperature; wherein signals to be generated by the two signal-generating means are not differentiated by a single type of detector; and

(c) determining the presence of at least one target nucleic acid sequence of the two target nucleic acid sequences by at least one of the reference values and the signals detected in the step (b).

According to conventional real-time PCR methods using amplification curves, it is common knowledge in the art that a plurality of target nucleic acid sequences cannot be differentially detected by use of signal-generating means providing undistinguishable identical signals.

The present invention overcomes limitations associated with the common knowledge in the art and leads to unexpected results to detect target nucleic acid sequences in greatly improved manner.

The present invention will be described in more detail as follows:

## Step (a): <u>Providing reference values</u>

The first reference value for the first target nucleic acid sequence and the second reference value for the second target nucleic acid sequence are provided.

Interestingly, the present inventors have found that when signals indicating the presence of a single target nucleic acid sequence are detected in a single reaction vessel at predetermined two detection temperatures, there is a signal change in a certain relationship (pattern or rule). For example, a signal change between a signal detected at the relatively high detection temperature and a signal detected at the relatively low detection temperature for a target nucleic acid sequence shows a certain relationship (pattern or rule). For example, the intensities of the signals may be identical or substantially identical to each other or the intensities of the signals may be different from each other but in a certain range at the two detection temperatures.

The feature of the present invention is to adopt the findings to obtaining reference values and detecting target nucleic acid sequences. Because signals for a target nucleic acid sequence in a single reaction vessel are detected with differing only detection temperatures (e.g. no change of amount of the target or no variation of buffer conditions), there is a certain relationship (pattern or rule) in a signal change between the two detection temperatures. Based on the certain relationship (pattern or rule) in the signal change, the signal detected at the relatively high detection temperature can be used for analyzing the signal detected at the relatively low detection temperature and vice versa. According to an embodiment, the present method is performed in a condition that permits a certain relationship (pattern or rule) in a signal change for a target nucleic acid sequence between the two detection temperatures.

"Reference value (RV)" of a target nucleic acid sequence represents a relationship of change in signals provided by a signal-generating means for detection of the target nucleic acid sequence means at two detection temperature.

According to an embodiment, each reference value is obtained by incubating a standard material corresponding to target nucleic acid sequence and signal-generating means, detecting signals at a relatively high detection temperature and a relatively low detection temperature, and then obtaining a difference between the signals detected at the relatively high detection temperature and the relatively low detection temperature.

According to an embodiment, (i) a first reference value for the first target nucleic acid sequence is obtained by (i-1) incubating the first target nucleic acid sequence with a first signal-generating means for detection of the first target nucleic acid sequence, (i-2) detecting signals at a relatively high detection temperature and a relatively low detection temperature, and (i-3) then obtaining a difference between the signals detected at the relatively high detection temperature and the relatively low detection temperature, and (ii) a second reference value for the second target nucleic acid sequence is obtained by (ii-1) incubating the second target nucleic acid sequence with a second signal-generating means for detection of the second target nucleic acid sequence, (ii-2) detecting signals at the relatively high detection temperature and the relatively low detection temperature, and (ii-3) then obtaining a difference between the signals detected at the relatively high detection temperature and the relatively low detection temperature; wherein the first reference value is different from the second reference value.

Each reference value may be provided in accordance with various manners.

According to an embodiment, each reference value is provided by experiments of a person performing the present method.

Alternatively, each reference value is provided by manufacturers of a kit, computer readable storage medium, a device or a computer program of this invention. The instructions packaged in the kit, computer readable storage medium product, a device or a computer program may contain reference values for target nucleic acid sequences of interest.

According to an embodiment, a reference value is obtained by mathematically processing the signals detected at the relatively high detection temperature and the signal detected at the relatively low detection temperature.

Such mathematical processing is a function of the signals. The function used in obtaining reference values include any function so long as it gives a relationship of change in signals provided by the signal-generating means at the relatively high detection temperature and the relatively low detection temperature. For instance, the function may be presented as a mathematical processing such as addition, multiplication, subtraction and division of signals.

The characteristics of the signals provided at the relatively high detection temperature and the relatively low detection temperature *per se* may be used to obtain a relationship of change in signals at the relatively high detection temperature and the relatively low detection temperature. Alternatively, the signals at the relatively high detection temperature and the relatively low detection temperature may be modified by mathematically processing the characteristics of the signals and used to obtain the relationship of change in signals at the relatively high detection temperature and the relatively low detection temperature.

Alternatively, the initially obtained reference value may be modified and used as a reference value.

According to an embodiment, the term "signal" with conjunction with the reference value includes not only signals *per se* obtained at detection temperatures but also a modified signal provided by mathematically processing the signals.

The reference value used in this invention may be obtained in various manners. For instance, the reference value may be given as an anticipated value. In considering a target sequence, a signal-generating means and detection temperatures, the reference value representing a relationship of change in signals at the relatively high detection temperature and the relatively low detection temperature may be anticipated.

The term "difference between signals detected at the first detection temperature and the second detection temperature" in obtaining a reference value is an embodiment of a relationship of change in signals at the first detection temperature and the second detection temperature.

According to an embodiment, the difference between the signals detected at the relatively high detection temperature and the relatively low detection temperature comprises a difference to be obtained by mathematically processing the signal detected at the relatively high detection temperature and the signal detected at the relatively low detection temperature.

According to an embodiment, where the mathematical processing is done, the characteristics of the signal should be vulnerable to the mathematical processing. In certain embodiment, the mathematical processing includes calculation (e.g., addition, multiplication, subtraction and division) using signals or obtaining other values derived from signals.

The difference between the signals at the relatively high detection temperature and the relatively low detection temperature may be expressed in various aspects. For example, the difference may be expressed as numerical values, the presence/absence of signal or plot with signal characteristics.

The mathematical processing of the signals for obtaining the difference may be carried out by various calculation methods and their modifications.

In particular, the mathematical processing of the signals for obtaining the difference may be carried out by calculating a ratio between signals at the relatively high detection temperature and the relatively low detection temperature, as claimed.

For instance, the ratio of the end-point intensity of the signal detected at the second detection temperature to the end-point intensity of the signal detected at the first detection temperature may be used as reference values.

According to an embodiment of this invention, the mathematical processing of the signals to obtain the difference between the signals is a calculation of a ratio of the signal detected at the relatively low detection temperature to the signal detected at the relatively high detection temperature. According to an embodiment of this invention, the mathematical processing of the signals to obtain the difference between the signals is a calculation of a ratio of the signal detected at the relatively high detection temperature to the signal detected at the relatively low detection temperature.

According to an embodiment, which is not claimed, a reference value may be obtained by calculating the subtraction between the signal detected at the relatively high detection temperature and the signal detected at the relatively low detection temperature.

The mathematical processing for obtaining the difference may be carried out in various fashions. The mathematical processing may be carried out by use of a machine. For example, the signals may undergo a mathematical processing by a processor in a detector or real-time PCR device. Alternatively, the signals may manually undergo a mathematical processing particularly according to a predetermined algorithm.

According to an embodiment of the present invention, the first reference value is different from the second reference value. According to an embodiment of the present invention, the first signal-generating means and the second signal-generating means are designed such that the first reference value is different from the second reference value.

Where the RV values are different from each other, a quantitative expression describing a difference extent may be varied depending on approaches for calculating the RV values.

According to an embodiment, signals for obtaining reference values may be processed by a common calculation method to provide a reference value for comparison, and then a difference extent between two reference values may be obtained by using the reference value for comparison. According to an embodiment, the common calculation method is division of two signals.

For instance, while two signals are processed by subtraction for obtaining reference values used to analyze signals according to the present method, the two signals may be processed by division for obtaining a reference value for comparison.

According to an embodiment of the present invention, the first reference value is at least 1.1-fold, 1.2-fold, 1.3-fold, 1.5-fold, 1.7-fold, 2-fold, 2.5-fold, 3-fold, 3.5-fold, 4-fold, 5-fold or 10-fold larger than the second reference value. According to an embodiment of the present invention, the second reference value is at least 1.1-fold, 1.2-fold, 1.3-fold, 1.5-fold, 1.7-fold, 2-fold, 2.5-fold, 3-fold, 3.5-fold, 4-fold, 5-fold or 10-fold larger than the first reference value.

According to an embodiment, where the comparison is performed to determine whether the first reference value is different from the second reference, the reference values are calculated by division of the signals. According to an embodiment, the method of calculating the reference value for determining whether the first reference value is different from the second reference may be the same or different from the method of calculating the reference value for detecting the target nucleic acid sequence.

According to an embodiment of this invention, signal-generating means for the reference value may be the same as that for the detection of the target nucleic acid sequence.

According to an embodiment, the incubation conditions for obtaining reference values are the same as those for analysis of the sample.

For a target nucleic acid sequence, the reference values may be obtained in various reaction conditions including the amount of component (e.g. the target nucleic acid sequence, signal-generating means, enzymes, or dNTPs), buffer pH or reaction time. According to an embodiment of this invention, the reference value may be obtained under reaction conditions sufficient to provide a saturated signal at the reaction completion. According to an embodiment of this invention, the difference between the signals obtained in obtaining the reference value has a certain range and the reference value is selected within the certain range or with referring to the certain range. According to an embodiment of this invention, the reference value may be selected with maximum or minimum value of the certain range or with referring to maximum or minimum value of the certain range. Particularly, the reference value may be modified in considering standard variation of the reference values obtained in various conditions, acceptable error ranges, specificity or sensitivity.

The present invention utilizes signal-generating means for providing signals for target nucleic acid sequences. Each of the target nucleic acid sequences is detected by a corresponding signal-generating means.

The term used herein "signal-generating means" refers to any material used in generation of signals indicating the presence of target nucleic acid sequences, for example including oligonucleotides, labels and enzymes. Alternatively, the term used herein "signal-generating means" can be used to refer to any methods using the materials for signal generation.

According to an embodiment of this invention, the incubation is preformed under conditions allowing a signal generation by the signal-generation means. Such conditions include temperatures, salt concentrations and pH of solutions.

Examples of the oligonucleotides serving as signal-generating means include oligonucleotides to be specifically hybridized with target nucleic acid sequences (e.g., probes and primers); where probes or primers hybridized with target nucleic acid sequences are cleaved to release a fragment, the oligonucleotides serving as signal-generating means include capture oligonucleotides to be specifically hybridized with the fragment; where the fragment hybridized with the capture oligonucleotide is extended to form an extended strand, the oligonucleotides serving as signal-generating means include oligonucleotides to be specifically hybridized with the extended strand; the oligonucleotides serving as signal-generating means include oligonucleotides to be specifically hybridized with the capture oligonucleotide; and the oligonucleotides serving as signal-generating means include combinations thereof.

While a signal generation principle is the same, the signal generating means comprising different sequences of oligonucleotides used may be considered different from each other.

The label may be linked to oligonucleotides or may be in the free form. The label may be incorporated into extended products during an extension reaction.

Where the cleavage of oligonucleotides is used in signal generation, examples of the enzyme include 5'-nuclease and 3'-nuclease, particularly nucleic acid polymerase having 5'-nuclease activity, nucleic acid polymerase having 3'-nuclease activity or FEN nuclease.

In the present invention, signals may be generated by using various materials described above in various fashions.

According to an embodiment, at least one of the two signal-generating means is a signal-generating means to generate a signal in a dependent manner on the formation of a duplex.

According to an embodiment, the signal-generating means for each of the target nucleic acid sequences are signal-generating means to generate a signal in a dependent manner on the formation of a duplex.

According to an embodiment, the duplex includes a double stranded target nucleic acid sequence.

The expression used herein "generate a signal in a dependent manner on the formation of a duplex" in conjunction with signal-generating means refers to that signal to be detected is provided being dependent on association or dissociation of two nucleic acid molecules. The expression includes that a signal is provided by a duplex *(e.g.* a detection oligonucleotide with a label and a nucleic acid sequence) formed being dependent on the presence of a target nucleic acid sequence. In addition, the expression includes that a signal is provided by inhibition of hybridization of a duplex *(e.g.* a detection oligonucleotide with a label and a nucleic acid sequence) wherein the inhibition is caused by the formation of another duplex.

Particularly, the signal is generated by the formation of a duplex between a target nucleic acid sequence and a detection oligonucleotide specifically hybridized with the target nucleic acid sequence.

The term used herein "detection oligonucleotide" is an oligonucleotide which is involved in generation of signal to be detected. According to an embodiment of the present invention, the detection oligonucleotide includes an oligonucleotide which is involved in an actual signal generation. For example, the hybridization or non-hybridization of a detection oligonucleotide to another oligonucleotide (e.g. a target nucleic acid sequence or an oligonucleotide comprising a nucleotide sequence complementary to the detection oligonucleotide) determines the signal generation.

According to an embodiment of the present invention, the detection oligonucleotide comprises at least one label.

The signal by the formation of a duplex between a target nucleic acid sequence and the detection oligonucleotide may be generated by various methods, including Scorpion method (Whitcombe et al, Nature Biotechnology 17:804-807 (1999)), Sunrise (or Amplifluor) method (Nazarenko et al, Nucleic Acids Research, 25(12):2516-2521 (1997), and U.S. Pat. No. 6,117,635), Lux method (U.S. Pat. No. 7,537,886), Plexor method (Sherrill C B, et al., Journal of the American Chemical Society, 126:4550-45569 (2004)), Molecular Beacon method (Tyagi et al, Nature Biotechnology v.14 MARCH 1996), HyBeacon method (French DJ et al., Mol. Cell Probes, 15(6):363-374(2001)), adjacent hybridization probe method (Bernard, P.S. et al., Anal. Biochem., 273:221(1999)) and LNA method (U.S. Pat. No. 6,977,295).

Particularly, the signal is generated by a duplex formed in a dependent manner on cleavage of a mediation oligonucleotide specifically hybridized with the target nucleic acid sequence.

The term used herein "mediation oligonucleotide" is an oligonucleotide which mediates production of a duplex not containing a target nucleic acid sequence.

According to an embodiment of the present invention, the cleavage of the mediation oligonucleotide *per se* does not generate signal and a fragment formed by the cleavage is involved in successive reactions for signal generation following hybridization and cleavage of the mediation oligonucleotide.

According to an embodiment, the hybridization or cleavage of the mediation oligonucleotide *per se* does not generate signal.

According to an embodiment of the present invention, the mediation oligonucleotide includes an oligonucleotide which is hybridized with a target nucleic acid sequence and cleaved to release a fragment, leading to mediate the production of a duplex. Particularly, the fragment mediates a production of a duplex by an extension of the fragment on a capture oligonucleotide.

According to an embodiment of the present invention, the mediation oligonucleotide comprises (i) a 3'-targeting portion comprising a hybridizing nucleotide sequence complementary to the target nucleic acid sequence and (ii) a 5'-tagging portion comprising a nucleotide sequence non-complementary to the target nucleic acid sequence.

According to an embodiment of the present invention, the cleavage of a mediation oligonucleotide release a fragment and the fragment is specifically hybridized with a capture oligonucleotide and extended on the capture oligonucleotide.

According to an embodiment of the present invention, a mediation oligonucleotide hybridized with target nucleic acid sequences is cleaved to release a fragment and the fragment is specifically hybridized with a capture oligonucleotide and the fragment is extended to form an extended strand, resulting in formation of a extended duplex between the extended stand and the capture oligonucleotide providing a signal indicating the presence of the target nucleic acid sequence.

According to an embodiment of the present invention, where a third oligonucleotide comprising a hybridizing nucleotide, sequence complementary to the extended strand is used, the hybridization of the third oligonucleotide and the extended strand forms other type of a duplex providing a signal indicating the presence of the target nucleic acid sequence.

According to an embodiment of the present invention, where a third oligonucleotide comprising a hybridizing nucleotide sequence complementary to the capture oligonucleotide is used, the formation of a duplex between the third oligonucleotide and the capture oligonucleotide is inhibited by the formation of the duplex between the extended strand and the capturing oligonucleotide, leading to provide a signal indicating the presence of the target nucleic acid sequence.

According to an embodiment of the present invention, the fragment, the extended strand, the capture oligonucleotide, the third oligonucleotide or combination of them can work as the detection oligonucleotide.

The signal by the duplex formed in a dependent manner on cleavage of the mediation oligonucleotide may be generated by various methods, including PTOCE (PTO cleavage and extension) method (WO 2012/096523), PCE-SH (PTO Cleavage and Extension-Dependent Signaling Oligonucleotide Hybridization) method (WO 2013/115442) and PCE-NH (PTO Cleavage and Extension-Dependent Non-Hybridization) method (PCT/KR2013/012312).

With referring to terms disclosed in the above references, the corresponding examples of the oligonucleotides are as follows: a mediation oligonucleotide corresponds to a PTO (Probing and Tagging Oligonucleotide), a capture oligonucleotide to a CTO (Capturing and Templating Oligonucleotide) and a third oligonucleotide to SO (Signaling Oligonucleotide) or HO (Hybridization Oligonucleotide). SO, HO, CTO, extended strand or their combination can play a role as a detection oligonucleotide.

The signal by the duplex formed in a dependent manner on cleavage of the mediation oligonucleotide includes the signal provided by inhibition of the formation of other duplex by the duplex formed in a dependent manner on cleavage of the mediation oligonucleotide (e.g. PCE-NH).

For example, where the signal by the duplex formed in a dependent manner on cleavage of the mediation oligonucleotide is generated by PTOCE method, the signal-generating means comprises an upstream oligonucleotide and a PTO (Probing and Tagging Oligonucleotide) comprising a hybridizing nucleotide sequence complementary to the target nucleic acid sequence, a CTO (Capturing and Templating Oligonucleotide), suitable label and a template-dependent nucleic acid polymerase having 5' nuclease activity. The PTO comprises (i) a 3'-targeting portion comprising a hybridizing nucleotide sequence complementary to the target nucleic acid sequence and (ii) a 5'-tagging portion comprising a nucleotide sequence non-complementary to the target nucleic acid sequence. The CTO comprises in a 3' to 5' direction (i) a capturing portion comprising a nucleotide sequence complementary to the 5'-tagging portion or a part of the 5'-tagging portion of the PTO and (ii) a templating portion comprising a nucleotide sequence non-complementary to the 5'-tagging portion and the 3'-targeting portion of the PTO.

The particular example of the signal generation by PTOCE method comprises the steps of:
(a) hybridizing the target nucleic acid sequence with the upstream oligonucleotide and the PTO; (b) contacting the resultant of the step (a) to an enzyme having a 5' nuclease activity under conditions for cleavage of the PTO; wherein the upstream oligonucleotide or its extended strand induces cleavage of the PTO by the enzyme having the 5' nuclease activity such that the cleavage releases a fragment comprising the 5'-tagging portion or a part of the 5'-tagging portion of the PTO; (c) hybridizing the fragment released from the PTO with the CTO; wherein the fragment released from the PTO is hybridized with the capturing portion of the CTO; and (d) performing an extension reaction using the resultant of the step (c) and a template-dependent nucleic acid polymerase; wherein the fragment hybridized with the capturing portion of the CTO is extended and an extended duplex is formed; wherein the extended duplex has a $T_m$ value adjustable by (i) a sequence and/or length of the fragment, (ii) a sequence and/or length of the CTO or (iii) the sequence and/or length of the fragment and the sequence and/or length of the CTO; wherein the extended duplex provides a target signal by (i) at least one label linked to the fragment and/or the CTO, (ii) a label incorporated into the extended duplex during the extension reaction, (iii) a label incorporated into the extended duplex during the extension reaction and a label linked to the fragment and/or the CTO, or (iv) an intercalating label; and (e) detecting the extended duplex by measuring the target signal at a predetermined temperature that the extended duplex maintains its double-stranded form, whereby the presence of the extended duplex indicates the presence of the target nucleic acid sequence. In this case, the method further comprises repeating all or some of the steps (a)-(e) with denaturation between repeating cycles.

In the phrase "denaturation between repeating cycles", the term "denaturation" means to separate a double-stranded nucleic acid molecule to a single-stranded nucleic acid molecule.

In the step (a) of PTOCE method, a primer set for amplification of the target nucleic acid sequence may be used instead of the upstream oligonucleotide. In this case, the method further comprises repeating all or some of the steps (a)-(e) with denaturation between repeating cycles.

The PTOCE method can be classified as a process in which the PTO fragment hybridized with the CTO is extended to form an extended strand and the extended strand is then detected. The PTOCE method is characterized that the formation of the extended strand is detected by using the duplex between the extended strand and the CTO.

There is another approach to detect the formation of the extended strand. For example, the formation of the extended strand may be detected by using an oligonucleotide specifically hybridized with the extended strand (e.g., PCE-SH method). In this method, the signal may be provided from (i) a label linked to the oligonucleotide specifically hybridized with the extended strand, (ii) a label linked to the oligonucleotide specifically hybridized with the extended strand and a label linked to the PTO fragment, (iii) a label linked to the oligonucleotide specifically hybridized with the extended strand and a label incorporated into the extended strand during the extension reaction, or (iv) a label linked to the oligonucleotide specifically hybridized with the extended strand and an intercalating dye. Alternatively, the signal may be provided from (i) a label linked to the extended strand or (ii) an intercalating dye.

Alternatively, the detection of the formation of the extended strand is performed by another method in which inhibition of the hybridization between the CTO and an oligonucleotide being specifically hybridizable with the CTO is detected(e.g. PCE-NH method). Such inhibition is considered to be indicative of the presence of a target nucleic acid sequence. The signal may be provided from (i) a label linked to the oligonucleotide being hybridizable with the CTO, (ii) a label linked to the CTO, (iii) a label linked to the oligonucleotide being hybridizable with the CTO and a label linked to the CTO, or (iv) an intercalating label.

According to an embodiment, the oligonucleotide being specifically hybridizable with the CTO has an overlapping sequence with the PTO fragment.

According to an embodiment, the detection oligonucleotide includes the oligonucleotide being specifically hybridizable with the extended strand (e.g., PCE-SH method) and oligonucleotide being specifically hybridizable with the CTO (e.g. PCE-NH method). According to an embodiment, the detection oligonucleotide includes the extended strand produced during a reaction or CTO.

The PTOCE-based methods commonly involve the formation of the extended strand depending on the presence of a target nucleic acid sequence. The term "PTOCE-based method" is used herein to intend to encompass various methods for providing signals comprising the formation of an extended strand through cleavage and extension of PTO.

The example of signal generation by the PTOCE-based methods comprises the steps of: (a) hybridizing the target nucleic acid sequence with the upstream oligonucleotide and the PTO; (b) contacting the resultant of the step (a) to an enzyme having a 5' nuclease activity under conditions for cleavage of the PTO; wherein the upstream oligonucleotide or its extended strand induces cleavage of the PTO by the enzyme having the 5' nuclease activity such that the cleavage releases a fragment comprising the 5'-tagging portion or a part of the 5'-tagging portion of the PTO; (c) hybridizing the fragment released from the PTO with the CTO; wherein the fragment released from the PTO is hybridized with the capturing portion of the CTO; (d) performing an extension reaction using the resultant of the step (c) and a template-dependent nucleic acid polymerase; wherein the fragment hybridized with the capturing portion of the CTO is extended to form an extended strand; and (e) detecting the formation of the extended strand by detecting signal generated dependent on the presence of the extended strand. In the step (a), a primer set for amplification of the target nucleic acid sequence may be used instead of the upstream oligonucleotide. In this case, the method further comprises repeating all or some of the steps (a)-(e) with denaturation between repeating cycles.

According to an embodiment, the signal generated by the formation of a duplex includes signals induced by hybridization of the duplex (e.g., hybridization of the duplex *per se,* or hybridization of a third oligonucleotide) or by inhibition of hybridization of a third oligonucleotide due to the formation of a duplex.

According to an embodiment, the signal-generating means for at least one of the target nucleic acid sequences is a signal-generating means by formation of a duplex in a dependent manner on cleavage of a mediation oligonucleotide specifically hybridized with the target nucleic acid sequence.

According to an embodiment, the signal-generating means for each of the target nucleic acid sequences are a signal-generating means by formation of a duplex in a dependent manner on cleavage of a mediation oligonucleotide specifically hybridized with the target nucleic acid sequence.

According to an embodiment, at least one of the two signal-generating means is a signal-generating means to generate a signal in a dependent manner on cleavage of a detection oligonucleotide.

According to an embodiment, both of the two signal-generating means are a signal-generating means to generate a signal in a dependent manner on cleavage of a detection oligonucleotide.

Particularly, the signal is generated by hybridization of the detection oligonucleotide with a target nucleic acid sequence and then cleavage of the detection oligonucleotide.

The signal by hybridization of the detection oligonucleotide with a target nucleic acid sequence and then cleavage of the detection oligonucleotide may be generated by various methods, including TaqMan probe method (U.S. Pat. Nos. 5,210,015 and 5,538,848).

Where the signal is generated by TaqMan probe method, the signal-generating means includes a primer set for amplification of a target nucleic acid sequence, TaqMan probe having a suitable label (e.g., interactive dual label) and nucleic acid polymerase having 5'-nuclease activity. The TaqMan probe hybridized with a target nucleic acid sequence is cleaved during target amplification and generates signal indicating the presence of the target nucleic acid sequence.

The particular example generating signal by TaqMan probe method comprises the step of: (a) hybridizing the primer set and TaqMan probe having a suitable label (e.g., interactive dual label) with the target nucleic acid sequence; (b) amplifying the target nucleic acid sequence by using the resultant of the step (a) and nucleic acid polymerase having 5'-nuclease activity, wherein the TaqMan probe is cleaved to release the label; and (c) detecting a signal generation from the released label.

Particularly, the signal is generated by cleavage of the detection oligonucleotide in a dependent manner on cleavage of a mediation oligonucleotide specifically hybridized with the target nucleic acid sequence.

According to an embodiment of the present invention, where a mediation oligonucleotide hybridized with target nucleic acid sequences is cleaved to release a fragment, the fragment is specifically hybridized with a detection oligonucleotide and the fragment induces the cleavage of the detection oligonucleotide.

According to an embodiment of the present invention, where a mediation oligonucleotide hybridized with target nucleic acid sequences is cleaved to release a fragment, the fragment is extended to cleave a detection oligonucleotide comprising a hybridizing nucleotide sequence complementary to the capture oligonucleotide.

According to an embodiment of the present invention, where a mediation oligonucleotide hybridized with target nucleic acid sequences is cleaved to release a fragment, the fragment is specifically hybridized with a detection oligonucleotide and the fragment induces the cleavage of the detection oligonucleotide.

According to an embodiment of the present invention, where a mediation oligonucleotide hybridized with target nucleic acid sequences is cleaved to release a fragment, the fragment is extended to cleave a detection oligonucleotide comprising a hybridizing nucleotide sequence complementary to the capture oligonucleotide.

The signal by cleavage of the detection oligonucleotide in a dependent manner on cleavage of the mediation oligonucleotide may be generated by various methods, including Invader assay (U.S. Pat. No. US 5,691,142), PCEC (PTO Cleavage and Extension-Dependent Cleavage) method (WO 2012/134195) and a method described in US Pat. No. 7,309,573. In particular, the method described in US Pat No. 7,309,573 may be considered as one of PTOCE-based methods using signal generation by cleavage, and in the method, the formation of the extended strand may be detected by detecting cleavage of an oligonucleotide specifically hybridized with the CTO by the formation of the extended strand. Invader assay forms a fragment by cleavage of a mediation oligonucleotide and induces successive cleavage reactions with no extension of the fragment.

According to an embodiment of the present invention, where the signal is generated in a dependent manner on cleavage of a detection oligonucleotide, the cleavage of the detection oligonucleotide induces signal changes or releases a labeled fragment to be detected.

Where a signal-generating means generates a signal by cleavage of a detection oligonucleotide as well as by the formation of a duplex, the signal-generating means may be considered as a signal generating means providing signal by cleavage, so long as it is used to generate signal by cleavage.

Even though the signal-generating means to generate signals in a dependent manner on cleavage of a detection oligonucleotide generates signals upon cleavage of the detection oligonucleotide, the signals detected at the relatively high detection temperature and the relatively low detection temperature are different from each other. The difference in the detected signals (e.g. difference in signal intensity) may be due to signal by hybridization of the detection oligonucleotide with a target nucleic acid sequence and/or temperature influence on signal generation of labels (*e.g.* dyes). Such difference is addressed in Examples using TaqMan probe method.

Interestingly, the present invention makes it practical to detect two target sequences by using different detection temperatures and signal-generating means to generate signals in a dependent manner on cleavage of a detection oligonucleotide (*e.g.* TaqMan probe method).

According to the embodiment of this invention, the signal-generating means for the target nucleic acid sequences are combination of a signal-generating means by cleavage of a detection oligonucleotide, and a signal-generating means by the formation of a duplex.

According to an embodiment, the detection oligonucleotide comprises at least one label.

According to an embodiment of the present invention, the detection oligonucleotide may be composed of at least one oligonucleotide. According to an embodiment of the present invention, where the detection oligonucleotide is composed of a plurality of oligonucleotides, it may have a label in various manners. For instance, one oligonucleotide among a plurality of oligonucleotides may have at least one label, a plurality of oligonucleotides all may have at least one label, or one portion of oligonucleotides may have at least one label and the other portion may not have a label.

The signals generated by the two signal-generating means are not differentiated by a single type of detector. The term "signals not differentiated by a single type of detector" means that signals are not differentiated from each other by a single type of detector due to their identical or substantially identical signal properties (*e.g.,* optical properties, emission wavelength and electrical signal). For example, where the same label (*e.g.,* FAM) is used for two target nucleic acid sequences and a single type of detector for detection of emission wavelength from FAM is used, signals are not differentially detected.

The term used herein "a single type of signal" means signals providing identical or substantially identical signal properties (e.g., optical properties, emission wavelength and electrical signal). For example, FAM and CAL Fluor 610 provide different types of signals.

The term used herein "a single type of detector" means a detection means for a singly type of signal. In a detector comprising several channels (e.g., photodiodes) for several different types of signals, each channel (e.g., a photodiode) corresponds to "a single type of detector".

According to an embodiment of this invention, the two signal-generating means comprise an identical label and signals from the label are not differentiated by the single type of detector.

The label useful in the present invention includes various labels known in the art. For example, the label useful in the present invention includes a single label, an interactive dual label, an intercalating dye and an incorporating label.

The single label includes, for example, a fluorescent label, a luminescent label, a chemiluminescent label, an electrochemical label and a metal label. According to an embodiment, the single label provides a different signal (e.g., different signal intensities) depending on its presence on a double strand or single strand. According to an embodiment, the single label is a fluorescent label. The preferable types and binding sites of single fluorescent labels used in this invention are disclosed U.S. Pat. Nos. 7,537,886 and 7,348,141, the teachings of which are incorporated herein by references in their entireties. For example, the single fluorescent label includes JOE, FAM, TAMRA, ROX and fluorescein-based label. The single label may be linked to oligonucleotides by various methods. For instance, the label is linked to probes through a spacer containing carbon atoms (*e.g.,* 3-carbon spacer, 6-carbon spacer or 12-carbon spacer).

As a representative of the interactive label system, the FRET (fluorescence resonance energy transfer) label system includes a fluorescent reporter molecule (donor molecule) and a quencher molecule (acceptor molecule). In FRET, the energy donor is fluorescent, but the energy acceptor may be fluorescent or non-fluorescent. In another form of interactive label systems, the energy donor is non-fluorescent, *e.g.,* a chromophore, and the energy acceptor is fluorescent. In yet another form of interactive label systems, the energy donor is luminescent, e.g. bioluminescent, chemiluminescent, electrochemiluminescent, and the acceptor is fluorescent. The interactive label system includes a dual label based on "on contact-mediated quenching" (Salvatore et al., Nucleic Acids Research, 2002 (30) no.21 e122 and Johansson et al., J. AM. CHEM. SOC 2002 (124) pp 6950-6956). The interactive label system includes any label system in which signal change is induced by interaction between at least two molecules (e.g. dye).

The reporter molecule and the quencher molecule useful in the present invention may include any molecules known in the art. Examples of those are: Cy2™ (506), YO-PRO™-1 (509), YOYO™-1 (509), Calcein (517), FITC (518), FluorX™ (519), Alexa™ (520), Rhodamine 110 (520), Oregon Green™ 500 (522), Oregon Green™ 488 (524), RiboGreen™ (525), Rhodamine Green™ (527), Rhodamine 123 (529), Magnesium Green™ (531), Calcium Green™ (533), TO-PRO™-1 (533), TOTO1 (533), JOE (548), BODIPY530/550 (550), Dil (565), BODIPY TMR (568), BODIPY558/568 (568), BODIPY564/570 (570), Cy3™ (570), Alexa™ 546 (570), TRITC (572), Magnesium Orange™ (575), Phycoerythrin R&B (575), Rhodamine Phalloidin (575), Calcium Orange™ (576), Pyronin Y (580), Rhodamine B (580), TAMRA (582), Rhodamine Red™ (590), Cy3.5™ (596), ROX (608), Calcium Crimson™ (615), Alexa™ 594 (615), Texas Red(615), Nile Red (628), YO-PRO™-3 (631), YOYO™-3 (631), R-phycocyanin (642), C-Phycocyanin (648), TO-PRO™-3 (660), TOTO3 (660), DiD DilC(5) (665), Cy5™ (670), Thiadicarbocyanine (671), Cy5.5 (694), HEX (556), TET (536), Biosearch Blue (447), CAL Fluor Gold 540 (544), CAL Fluor Orange 560 (559), CAL Fluor Red 590 (591), CAL Fluor Red 610 (610), CAL Fluor Red 635 (637), FAM (520), Fluorescein (520), Fluorescein-C3 (520), Pulsar 650 (566), Quasar 570 (667), Quasar 670 (705) and Quasar 705 (610). The numeric in parenthesis is a maximum emission wavelength in nanometer. Preferably, the reporter molecule and the quencher molecule include JOE, FAM, TAMRA, ROX and fluorescein-based label.

Suitable fluorescence molecule and suitable pairs of reporter-quencher are disclosed in a variety of publications as follows: Pesce et al., editors, Fluorescence Spectroscopy (Marcel Dekker, New York, 1971); White et al., Fluorescence Analysis: A Practical Approach (Marcel Dekker, New York, 1970); Berlman, Handbook of Fluorescence Spectra of Aromatic Molecules, 2nd Edition (Academic Press, New York, 1971); Griffiths, Color AND Constitution of Organic Molecules (Academic Press, New York, 1976); Bishop, editor, Indicators (Pergamon Press, Oxford, 1972); Haugland, Handbook of Fluorescent Probes and Research Chemicals (Molecular Probes, Eugene, 1992); Pringsheim, Fluorescence and Phosphorescence (Interscience Publishers, New York, 1949); Haugland, R. P., Handbook of Fluorescent Probes and Research Chemicals, 6th Edition (Molecular Probes, Eugene, Oreg., 1996) U.S. Pat. Nos. 3,996,345 and 4,351,760.

It is noteworthy that a non-fluorescent quencher molecule (e.g. black quencher or dark quencher) capable of quenching a fluorescence of a wide range of wavelengths or a specific wavelength may be used in the present invention.

In the signaling system comprising the reporter and quencher molecules, the reporter encompasses a donor of FRET and the quencher encompasses the other partner (acceptor) of FRET. For example, a fluorescein dye is used as the reporter and a rhodamine dye as the quencher.

The interactive dual label may be linked to one strand of a duplex. Where the strand containing the interactive dual label leaves in a single stranded state, it forms a hairpin or random coil structure to induce quenching between the interactive dual label. Where the strand forms a duplex, the quenching is relieved. Alternatively, where the interactive dual label is linked to nucleotides adjacently positioned on the strand, the quenching between the interactive dual label occurs. Where the strand forms a duplex and then is cleaved, the quenching becomes relieved.

Each of the interactive dual label may be linked to each of two strands of the duplex. The formation of the duplex induces quenching and denaturation of the duplex induces unquenching. Alternatively, where one of the two stands is cleaved, the unquenching may be induced.

Exemplified intercalating dyes useful in this invention include SYBR™ Green I, PO-PRO™-1, BO-PRO™-1, SYTO™43, SYTO™44, SYTO™45, SYTOX™Blue, POPO™-1, POPO™-3, BOBO™-1, BOBO™-3, LO-PRO™-1, JO-PRO™-1, YO-PRO™1, TO-PRO™1, SYTO™11, SYTO™13, SYTO™15, SYTO™16, SYTO™20, SYTO™23, TOTO™-3, YOYO™3, GelStar™ and thiazole orange. The intercalating dyes intercalate specifically into double-stranded nucleic acid molecules to generate signals.

The incorporating label may be used in a process to generate signals by incorporating a label during primer extension (e.g., Plexor method, Sherrill C B, et al., Journal of the American Chemical Society, 126:4550-45569(2004)). The incorporating label may be also used in a signal generation by a duplex formed in a dependent manner on cleavage of a mediation oligonucleotide hybridized with the target nucleic acid sequence.

The incorporating label may be generally linked to nucleotides. The nucleotide having a non-natural base may be also used.

The term used herein "non-natural base" refers to derivatives of natural bases such as adenine (A), guanine (G), thymine (T), cytosine (C) and uracil (U), which are capable of forming hydrogen-bonding base pairs. The term used herein "non-natural base" includes bases having different base pairing patterns from natural bases as mother compounds, as described, for example, in U.S. Pat. Nos. 5,432,272, 5,965,364, 6,001,983, and 6,037,120. The base pairing between non-natural bases involves two or three hydrogen bonds as natural bases. The base pairing between non-natural bases is also formed in a specific manner. Specific examples of non-natural bases include the following bases in base pair combinations: iso-C/iso-G, iso-dC/iso-dG, K/X, H/J, and M/N (see U.S. Pat. No. 7,422,850).

Where the signal is generated by the PTOCE method, a nucleotide incorporated during the extension reaction may have a first non-natural base and the CTO may have a nucleotide having a second non-natural base with a specific binding affinity to the first non-natural base.

The term used herein "target - nucleic acid", "target nucleic acid sequence" or "target sequence" refers to a nucleic acid sequence of interest for detection or quantification. The target nucleic acid sequence comprises a sequence in a single strand as well as in a double strand. The target nucleic acid sequence comprises a sequence initially present in a nucleic acid sample as well as a sequence newly generated in reactions.

The target nucleic acid sequence may include any DNA (gDNA and cDNA), RNA molecules their hybrids (chimera nucleic acid). The sequence may be in either a double-stranded or single-stranded form. Where the nucleic acid as starting material is double-stranded, it is preferred to render the two strands into a single-stranded or partially single-stranded form. Methods known to separate strands includes, but not limited to, heating, alkali, formamide, urea and glycoxal treatment, enzymatic methods (e.g., helicase action), and binding proteins. For instance, strand separation can be achieved by heating at temperature ranging from 80°C to 105°C. General methods for accomplishing this treatment are provided by Joseph Sambrook, et al., Molecular Cloning, A Laboratory Manual, Cold Spring Harbor Laboratory Press, Cold Spring Harbor, N.Y.(2001).

Where a mRNA is employed as starting material, a reverse transcription step is necessary prior to performing annealing step, details of which are found in Joseph Sambrook, et al., Molecular Cloning, A Laboratory Manual, Cold Spring Harbor Laboratory Press, Cold Spring Harbor, N.Y.(2001); and Noonan, K. F. et al., Nucleic Acids Res. 16:10366 (1988)). For reverse transcription, an oligonucleotide dT primer hybridizable to poly A tail of mRNA, random primers or target-specific primers may be used.

The target nucleic acid sequence includes any naturally occurring prokaryotic, eukaryotic (for example, protozoans and parasites, fungi, yeast, higher plants, lower and higher animals, including mammals and humans), viral (for example, Herpes viruses, HIV, influenza virus, Epstein-Barr virus, hepatitis virus, polio virus, etc.), or viroid nucleic acid. The nucleic acid molecule can also be any nucleic acid molecule which has been or can be recombinantly produced or chemically synthesized. Thus, the nucleic acid sequence may or may not be found in nature. The target nucleic acid sequence may include known or unknown sequences.

### Step (b): Incubating samples with signal-generating means and signal detection

The sample to be analyzed is incubated with the first signal-generating means and the second signal-generating means for detection of the two target nucleic acid sequences and signals from the two signal-generating means are detected at the relatively high detection temperature and the relatively low detection temperature.

According to an embodiment, which is not claimed, each of signal-generating means for detection of each corresponding target nucleic acid sequences is designed such that signals are generated at both of the relatively high detection temperature and the relatively low detection temperature where the corresponding target nucleic acid sequence are present.

The two signal-generating means generate signals at the relatively high detection temperature and the relatively low detection temperature and signals to be generated by the two signal-generating means are not differentiated by a single type of detector.

According to an embodiment, the incubation conditions for analysis of the sample are the same as those for obtaining the first reference value and the second reference value.

Signals include various signal characteristics from the signal detection, *e.g.*, signal intensity [*e.g.*, RFU (relative fluorescence unit) value or in the case of performing amplification, RFU values at a certain cycle, at selected cycles or at end-point], signal change shape (or pattern) or $C_t$ value, or values obtained by mathematically processing the characteristics.

According to an embodiment, the term "signal" with conjunction with reference value or sample analysis includes not only signals *per se* obtained at detection temperatures but also a modified signal provided by mathematically processing the signals.

According to an embodiment of this invention, when an amplification curve is obtained by real-time PCR, various signal values (or characteristics) from the amplification curve may be selected used for determination of target presence (intensity, $C_t$ value or amplification curve data).

According to an embodiment, the signals used for the presence of target nucleic acid sequences are a significant signal. In other words, the signals are signal to be generated being dependent on the presence of target nucleic acid sequences. According to an embodiment, significance of signals detected may be determined using a threshold value. For example, a threshold value is predetermined from a negative control in considering background signals of detector, sensitivity or label used, and then the significance of signals may be determined.

According to an embodiment of this invention, the step (b) is performed in a signal amplification process concomitantly with a nucleic acid amplification. According to an embodiment of this invention, wherein the step (b) is performed in a signal amplification process without a nucleic acid amplification.

In the present invention, the signal generated by signal-generating means may be amplified simultaneously with target amplification. Alternatively, the signal may be amplified with no target amplification.

According to an embodiment of this invention, the signal generation is performed in a process involving signal amplification together with target amplification.

According to an embodiment of this invention, the target amplification is performed in accordance with PCR (polymerase chain reaction). PCR is widely employed for target amplification in the art, including cycles of denaturation of a target sequence, annealing (hybridization) between the target sequence and primers and primer extension (Mullis et al. U.S. Pat. Nos. 4,683,195, 4,683,202 and 4,800,159; Saiki et al., (1985) Science 230, 1350-1354). The signal may be amplified by applying the signal generation methods described above (*e.g.,* TaqMan method and PTOCE-based methods) to the PCR process. According to an embodiment, the present invention provides signals by real-time PCR method. According to an embodiment, the amplification of the target nucleic acid sequence is performed by PCR (polymerase chain reaction), LCR (ligase chain reaction, see Wiedmann M, et al., "Ligase chain reaction (LCR)- overview and applications." PCR Methods and Applications 1994 Feb;3(4):S51-64), GLCR (gap filling LCR, see WO 90/01069, EP 439182 and WO 93/00447), Q-beta (Q-beta replicase amplification, see Cahill P, et al., Clin Chem., 37(9):1482-5(1991), U.S. Pat. No. 5556751), SDA (strand displacement amplification, see G T Walker et al., Nucleic Acids Res. 20(7):16911696(1992), EP 497272), NASBA (nucleic acid sequence-based amplification, see Compton, J. Nature 350(6313):912(1991)), TMA (Transcription-Mediated Amplification, see Hofmann WP et al., J Clin Virol. 32(4):289-93(2005); U.S. Pat. No. 5888779).) or RCA (Rolling Circle Amplification, see Hutchison C.A. et al., Proc. Natl Acad. Sci. USA. 102:1733217336(2005)).

The amplification methods described above may amplify target sequences through repeating a series of reactions with or without changing temperatures. The unit of amplification comprising the repetition of a series of reactions is expressed as a "cycle". The unit of cycles may be expressed as the number of the repetition or time being dependent on amplification methods.

For example, the detection of signals may be performed at each cycle of amplification, selected several cycles or end-point of reactions. According to an embodiment, where signals are detected at at least two cycles, the detection of signal in an individual cycle may be performed at all detection temperatures or some selected detection temperatures. According to an embodiment of this invention, the detection is performed at the relatively high detection temperature in odd numbered cycles and at the relatively high detection temperature in even numbered cycles.

According to an embodiment of this invention, incubation is preformed in the conditions allowing target amplification well as signal generation by the signal-generation means.

The amplification of the target nucleic acid sequence is accomplished by target amplification means including a primer set for amplification and nucleic acid polymerase.

According to an embodiment of the present invention, a nucleic acid polymerase having a nuclease activity (*e.g.* 5' nuclease activity or 3' nuclease activity) may be used. According to an embodiment of the present invention, a nucleic acid polymerase having a no nuclease activity may be used.

The nucleic acid polymerase useful in the present invention is a thermostable DNA polymerase obtained from a variety of bacterial species, including *Thermus aquaticus* (Taq), *Thermus thermophilus* (Tth), *Thermus filiformis, Thermis flavus, Thermococcus literalis, Thermus antranikianii, Thermus caldophilus, Thermus chliarophilus, Thermus flavus, Thermus igniterrae, Thermus lacteus, Thermus oshimai, Thermus ruber, Thermus rubens, Thermus scotoductus, Thermus silvanus, Thermus species Z05, Thermus species sps 17, Thermus thermophilus, Thermotoga maritima, Thermotoga neapolitana, Thermosipho africanus, Thermococcus litoralis, Thermococcus barossi, Thermococcus gorgonarius, Thermotoga maritima, Thermotoga neapolitana, Thermosipho africanus, Pyrococcus woesei, Pyrococcus horikoshii, Pyrococcus abyssi, Pyrodictium occultum, Aquifex pyrophilus* and *Aquifex aeolieus.* Particularly, the thermostable DNA polymerase is Taq polymerase.

According to an embodiment of the present invention, the amplification of the target nucleic acid sequence is accomplished by an asymmetric PCR. The ratio of primers may be selected in consideration of cleavage or hybridization of downstream oligonucleotides.

According to an embodiment of this invention, the step (a) and/or step (b) is performed in a signal amplification process without a nucleic acid amplification.

Where the signal is generated by methods including cleavage of an oligonucleotide, the signal may be amplified with no target amplification. For example, the step (a) and/or step (b) ay be performed with amplification of signals but with no amplification of target sequences in accordance with CPT method (Duck P, et al., Biotechniques, 9:142-148 (1990)), Invader assay (U.S. Pat. Nos. 6,358,691 and 6,194,149), PTOCE-based methods (e.g., PCE-SH method, PCE-NH method and PCEC method) or CER method (WO 2011/037306).

The signal amplification methods described above may amplify signals through repeating a series of reactions with or without changing temperatures. The unit of signal amplification comprising the repetition of a series of reactions is expressed as a "cycle". The unit of cycles may be expressed as the number of the repetition or time being dependent on amplification methods.

For example, the generation and detection of signals may be performed at each cycle of amplification, selected several cycles or end-point of reactions.

During or after the incubation (reaction) of the sample with two signal-generating means to generate signal, the generated signal may be detected by using a single type of detector.

According to an embodiment, the detection temperatures for target nucleic acid sequences are predetermined in considering a temperature range to allow signal generation by the signal-generating means.

The present invention uses that there is a certain temperature range to allow signal generation in a dependent manner on signal-generating means.

For example, when a signal-generating means generates a signal upon hybridization (or association) between two nucleic acid molecules and no signal upon non-hybridization (or dissociation) between them, the signal is generated at temperatures allowing hybridization between two nucleic acid molecules, however, no signal is generated at temperatures failing to hybridize between two nucleic acid molecules. As such, there is a certain temperature range to allow signal generation (*i.e.,* signal detection) and other temperature range not to allow signal generation. The temperature ranges are affected by the $T_m$ value of the hybrid of the two nucleic acid molecules employed in the signal-generation means.

Where the signal generation method using a released fragment with a label after cleavage is employed, the signal may be theoretically detected at any temperature (*e.g.*, 30-99°C).

A detection temperature is selected from the temperature range to allow signal generation by the signal generation mean.

According to an embodiment, the signal-generating means for detecting a target nucleic acid sequence may be signal-generating means to provide different signals (*e.g.* signal intensity) from each other at the two detection temperatures.

According to an embodiment, when the signal-generating means to generate signals in a dependent manner on cleavage of a detection oligonucleotide (e.g. TaqMan probe method) is employed, signals from the signal-generating means may be different depending on detection temperatures in the sense that signal generation by hybridization of detection oligonucleotides with target nucleic acid sequences and signal generation from dyes may be affected by temperatures. For instance, a probe labeled with a fluorescent reporter molecule and a quencher molecule may generate different signals depending on detection temperatures in which the probe may generate higher signals at the relatively low detection temperature than those at the relatively high detection temperature.

In this regard, the present invention makes it practical to detect two target sequences by using different detection temperatures and signal-generating means to generate signals in a dependent manner on cleavage of a detection oligonucleotide. According to the embodiment of this invention, both of the two signal-generating means are a signal-generating means by cleavage of a detection oligonucleotide.

A detection temperature is selected from the temperature range to allow signal generation by the signal generation means. The term "the detection temperature range" is used herein to particularly describe the temperature range to allow signal generation (*i.e.,* signal detection).

According to the present invention, a temperature for detecting the presence of each of target nucleic acid sequences may be allocated in considering signal-generating means.

According to an embodiment, the relatively high detection temperature and the relatively low detection temperature at which the detection is carried out may be predetermined. For example, the relatively high detection temperature and the relatively low detection temperature are predetermined as 72°C and 60°C, respectively, and then signal-generating means suitable for the detection temperatures are constructed.

According to an embodiment of this invention, when the signal-generating means generates a signal in a dependent manner on the formation of a duplex, the detection temperature is selected based on a $T_m$ value of the duplex.

According to an embodiment of this invention, when the signal-generating means generates a signal in a dependent manner on the formation of a duplex, the detection temperature is controllable by adjusting a $T_m$ value of the duplex.

For example, where the signal is generated by a detection oligonucleotide specifically hybridized with the target nucleic acid sequence (*e.g.,* Lux probe, Molecular Beacon probe, HyBeacon probe and adjacent hybridization probe), the detection of the signal is successfully done at the predetermined temperature by adjusting the $T_m$ value of the oligonucleotide. Where Scorpion primer is used, the detection of the signal is successfully done at the predetermined temperature by adjusting the $T_m$ value of a portion to be hybridized with extended strand.

Where the signal is generated by the duplex formed dependent on the presence of the target nucleic acid sequence, the detection of the signal is successfully done at the predetermined temperature by adjusting the $T_m$ value of the duplex. For example, where the signal is generated by the PTOCE method, the detection of the signal is successfully done at the predetermined temperature by adjusting the $T_m$ value of the extended duplex formed by the extension of the PTO fragment on the CTO.

The PTOCE-based methods have advantages to readily adjust $T_m$ values of the duplex or a third hybrid whose hybridization is affected by the duplex.

According to an embodiment of this invention, when the signal-generating means generates a signal in a dependent manner on cleavage of a detection oligonucleotide, the detection temperature may be selected based on a $T_m$ value of the detection oligonucleotide because hybridization of detection oligonucleotides with target nucleic acid sequences induces signal generation even though the label released by the cleavage generates signals.

The detector used in the present invention includes any means capable of detecting signals. For example, where fluorescent signals are used, photodiodes suitable in detection of the fluorescent signals may be employed as detectors. The detection using a single type of detector means that the detection is performed by using a detector capable of single type of signal or using each channel (*i.e.,* photodiode) of a detector carrying several channels (*i.e.,* photodiodes).

According to an embodiment, the generation of signals includes "signal generation or extinguishment" and "signal increase or decrease" from labels.

The term used herein "sample" includes biological samples (*e.g.,* cells, tissues, and fluid from a biological source) and non-biological samples (e.g., food, water and soil). The biological samples includes, not limited to, virus, bacteria, tissue, cell, blood, serum, plasma, lymph, sputum, swab, aspirate, bronchoalveolar lavage fluid, milk, urine, feces, ocular fluid, saliva, semen, brain extracts, spinal cord fluid (SCF), appendix, spleen and tonsillar tissue extracts, amniotic fluid and ascitic fluid. In addition, the sample may include natural-occurring nucleic acid molecules isolated from biological sources and synthetic nucleic acid molecules.

It would be obvious to one of skill in the art that the step (b) may be carried out before performing the step (a). Accordingly, it is to be understood that such variants and modifications falls within the scope of the present invention determined by appended claims and their equivalents

### Step (c): Determination of the presence of target nucleic acid sequence by reference value and signal

Finally, the presence of at least one target nucleic acid sequence of the two target nucleic acid sequences is determined by at least one of the reference values and the signals detected in the step (b).

According to an embodiment, the present method is used for detection of two target nucleic acid sequences by the two reference values (*i.e.,* the first reference value and the second reference value).

The term used herein "by the reference values and the signals" with conjunction to determination of the presence of a target nucleic acid sequence means that the presence of a target nucleic acid sequence is determined by directly or indirectly using , modifying or mathematically processing the reference value provided in the step (a) and the signals generated from the signal-generating means, including using numerical values of the reference value and signals or their modifications, using ranges of reference value, plotting reference value and signal and using the presence/absence of signals. There is no intended distinction between the terms "by the reference value and the signals" and "by using the reference value and the signals", and these terms will be used interchangeably.

The presence of at least one of the two target nucleic acid sequences may be determined by using at least one of the two reference values provided in the step (a) and the signals detected in the step (b) such that more accurate determination is made as follows:

According to an embodiment, the presence of the first target nucleic acid sequence in the sample is determined as defined in the appended claims, by the second reference value and the signals detected in the step (b) at the relatively high detection temperature and the relatively low detection temperature, and the presence of the second target nucleic acid sequence in the sample is determined by the first reference value and the signals detected in the step (b) at the relatively high detection temperature and the relatively low detection temperature.

According to an embodiment, which is not claimed, the presence of the first target nucleic acid sequence in the sample is determined by a difference calculated with the second reference value and the signals detected in the step (b) and the presence of the second target nucleic acid sequence in the sample is determined by a difference calculated with the first reference value and the signals detected in the step (b).

In more particular, the determination of the presence of the first target nucleic acid sequence, as defined in the appended claims, comprises processing the second reference value and the signals detected in the step (b) to eliminate a signal generated by the second signal generating means and to determine generation of a signal by the first signal generating means; and the determination of the presence of the second target nucleic acid sequence comprises processing the first reference value and the signals detected in the step (b) to eliminate a signal generated by the first signal generating means and to determine generation of a signal by the second signal generating means.

In much more particular, the elimination of the signal generated by the second signal generating means is to mathematically eliminate the signal generated by the second signal generating means from the signals detected in the step (b) and the elimination of the signal generated by the first signal generating means is to mathematically eliminate the signal generated by the first signal generating means from the signals detected in the step (b).

In still much more particular, the signal generated at the relatively low detection temperature by the second signal generating means is eliminated from the signal detected at the relatively low detection temperature by the second reference value and the signal detected at the relatively high detection temperature, thereby determining whether the first signal generating means generates a signal at the relatively low detection temperature, which demonstrates the presence or absence of the first target nucleic acid sequence.

For example, where the second reference value is obtained by calculation of ratio between signals provided by the second signal-generating means at the two detection temperature, the generation of the signal at the relatively low detection temperature by the first signal generating means may be determined by subtracting a value from the signal detected at the relatively low detection temperature; wherein the value is obtained by multiplying or dividing the signal detected at the relatively high detection temperature by the second reference value. However, the scope of the claims is characterise by the calculation as defined in the claims, only.

According to an embodiment, whether "multiplying" or "dividing" of the signal detected at a detection temperature by a reference value is dependent on the method calculating the ratio, wherein the claims as characterised by the method as defined in the claims, only.

In still much more particular, the signal generated at the relatively high detection temperature by the second signal generating means is eliminated from the signal detected at the relatively high detection temperature by the second reference value and the signal detected at the relatively low detection temperature, thereby determining whether the first signal generating means generates a signal at the relatively high detection temperature, which demonstrates the presence or absence of the first target nucleic acid sequence.

For example, where the second reference value is obtained by calculation of ratio between signals provided by the second signal-generating means at the two detection temperatures, the generation of the signal at the relatively high detection temperature by the first signal generating means may be determined by subtracting a value from the signal detected at the relatively high detection temperature; wherein the value is obtained by multiplying or dividing the signal detected at the relatively low detection temperature by the second reference value. However, the scope of the claims is characterise by the calculation as defined in the claims, only.

In still much more particular, the signal generated at the relatively low detection temperature by the first signal generating means is eliminated from the signal detected at the relatively low detection temperature by the first reference value and the signal detected at the relatively high detection temperature, thereby determining whether the second signal generating means generates a signal at the relatively low detection temperature is determined.

For example, where the first reference value is obtained by calculation of ratio between signals provided by the first signal-generating means at the two detection temperature, generation of the signal at the relatively low detection temperature by the second signal generating means may be determined by subtracting a value from the signal detected at the relatively low detection temperature; wherein the value is obtained by multiplying or dividing the signal detected at the relatively high detection temperature by the first reference value. However, the scope of the claims is characterise by the calculation as defined in the claims, only.

In still much more particular, the signal generated at the relatively high detection temperature by the first signal generating means is eliminated from the signal detected at the relatively high detection temperature by the first reference value and the signal detected at the relatively low detection temperature, thereby determining whether the second signal generating means generates a signal at the relatively high detection temperature is determined.

For example, where the first reference value is obtained by calculation of ratio between signals provided by the first signal-generating means at the two detection temperature, generation of the signal at the relatively high detection temperature by the second signal generating means may be determined by subtracting a value from the signal detected at the relatively high detection temperature; wherein the value is obtained by multiplying or dividing the signal detected at the relatively low detection temperature by the first reference value. However, the scope of the claims is characterise by the calculation as defined in the claims, only.

The performance principle underlying the present invention will be described with reference to Example 1 as follows. However, the scope of the claims is characterised by the steps and the technical features defined in the claims, only.

In Example 1, the first target nucleic acid sequence (NG) and the first signal-generating means are incubated and signals at a relatively, low detection temperature (L) and a relatively high detection temperature (H) are then measured. The ratio of the detected signal ($FT_L$) at the relatively low detection temperature provided by the first signal-generating means to the detected signal ($FT_H$) at the relatively high detection temperature provided by the first signal-generating means is calculated and in turn used as the first reference value for the first target nucleic acid sequence ($RV$ of $NG = RV_F = (FT_L) \div (FT_H) = 1.8$).

The second target nucleic acid sequence (CT) and the second signal-generating means are incubated and signals at a relatively low detection temperature (L) and a relatively high detection temperature (H) are then measured. The ratio of the detected signal ($ST_L$) at the relatively low detection temperature provided by the second signal-generating means to the detected signal ($ST_H$) at the relatively high detection temperature provided by the second signal-generating means is calculated and in turn used as the second reference value for the second target nucleic acid sequence ($RV$ of $CT = RV_S = (ST_L) \div (ST_H) = 5.8$).

Where a sample is incubated with a first signal-generating means and a second signal-generating means, the fluorescent signals detected at the relatively high detection temperature and the relatively low detection temperature are represented as $F_H$ and $F_L$, respectively.

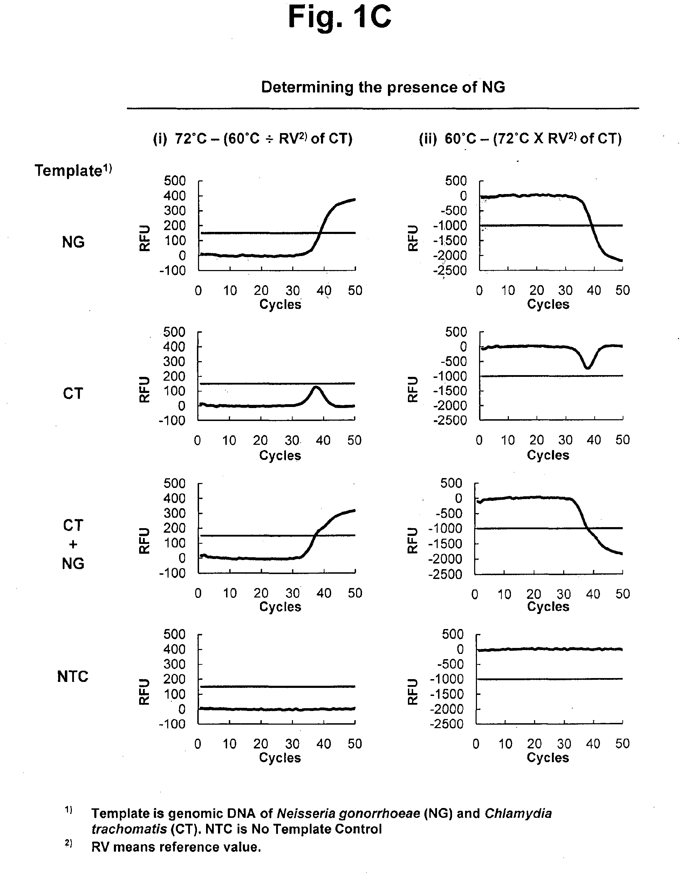

As Example 1, where the reference values for, target nucleic acid sequences are provided by a ratio, the following equation may be presented for determining whether the first signal generating means generates a signal at the relatively low detection temperature (see Fig. 1C and (ii)): $F_L - [F_H \times RV_S]$.

$F_H$ in the sample may be expressed as the sum of signals from the first target nucleic acid sequence ($FT_H$) and the second target nucleic acid sequence ($ST_H$) at the relatively high detection temperature, and $F_L$ in the sample may be expressed as the sum of signals from the first target nucleic acid sequence ($FT_L$) and the second target nucleic acid sequence ($ST_L$) at the relatively low detection temperature: $F_H = FT_H + ST_H$ and $F_L = FT_L + ST_L$.

$$F_L - [F_H \times RV_S]$$

may be expressed as follows:

$$F_L - [F_H \times RV_S] = (FT_L + ST_L) - [(FT_H + ST_H) \times RV_S]$$

$$= FT_L - RV_S \times FT_H + ST_L - RV_S \times ST_H$$

$$= FT_L - 5.8 \times FT_H + ST_L - 5.8 \times ST_H.$$

Where the sample comprises the second target nucleic acid sequence, ($ST_L$ -5.8 x $ST_H$) may substantially show the value of zero (0) because $RV_S = ST_L / ST_H = 5.8$.

Even where the second target nucleic acid sequence is not present in the sample, ($ST_L$ - 5.8 x $ST_H$) may substantially show the value of zero (0) because $ST_L$ and $ST_H$ are substantially value of zero (0).

Where the sample comprises the first target nucleic acid sequence, ($FT_L$ - 5.8 x $FT_H$) will show a negative value because the reference value of 5.8 is used in ($FT_L$ - 5.8 x $FT_H$) while the first reference value of the first target nucleic acid is 1.8.

Accordingly, where ($FT_L$ - 5.8 x $FT_H$) shows a negative value, the sample is determined to comprise the first target nucleic acid sequence (see Fig. 1C).

Where the first target nucleic acid sequence is not present in the sample, ($FT_L$ - 5.8 x $FT_H$) may substantially show the value of zero (0) (see Fig. 1C).

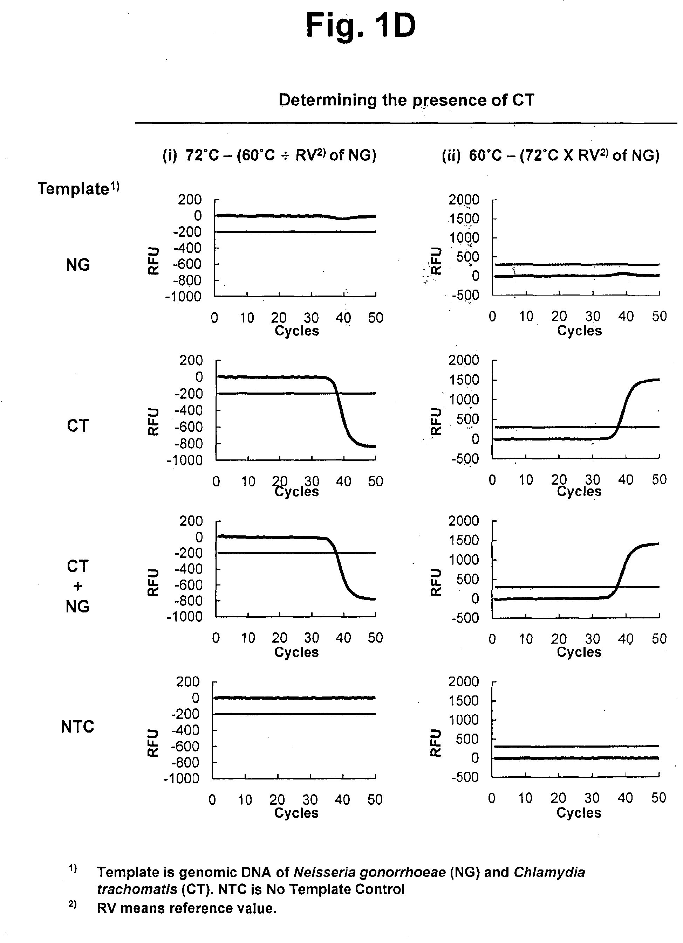

Alternatively, the following equation may be also presented for determining the presence of the second target nucleic acid sequence in the sample, *i.e.,* whether the second signal generating means generates a signal at the relatively low detection temperature (see Fig 1D and (ii)): $F_L - [F_H \times RV_F]$.

$$F_L - [F_H \times RV_F]$$

may be expressed as follows:

$$F_L - [F_H \times RV_F] = (FT_L + ST_L) - [(FT_H + ST_H) \times RV_F]$$

$$= FT_L - RV_F \times FT_H + ST_L - RV_F \times ST_H$$

$$= FT_L - 1.8 \times FT_H + ST_L - 1.8 \times ST_H.$$

Where the sample comprises the fist target nucleic acid sequence, $FT_L$ - 1.8 x $FT_H$ may substantially show the value of zero (0) because $RV_F = FT_L / FT_H = 1.8$.

Even where the first target nucleic acid sequence is not present in the sample, ($FT_L$ - 1.8 x $FT_H$) may substantially show the value of zero (0) because $FT_L$ and $FT_H$ are substantially value of zero (0).

Where the sample comprises the second target nucleic acid sequence, ($ST_L$ -1.8 x $ST_H$) will show a positive value because the reference value of 1.8 is used in ($ST_L$ - 1.8 x $ST_H$) while the second reference value for the second target nucleic acid is 5.8.

Accordingly, where ($ST_L$ - 1.8 x $ST_H$) shows a positive value, the sample is determined to comprise the second target nucleic acid sequence (see Fig. 1D).

Where the second target nucleic acid sequence is not present in the sample, ($ST_L$ - 1.8 x $ST_H$) may substantially show the value of zero (0) (see Fig. 1D).

In considering the principle for detection of a target nucleic acid sequence by analyzing signals at the relatively low detection temperature described above, detection of a target nucleic acid sequence may be accomplished by analyzing signals at a relatively high detection temperature as follows:
For example, the following equation may be presented for determination whether the first signal generating means generates a signal at the relatively high detection temperature (see Fig. 1C and (i)): $F_H - [F_L \div RV_S]$.

Alternatively, the following equation may be also presented for determining the presence of the second target nucleic acid sequence in the sample, *i.e.,* whether the second signal generating means generates a signal at the relatively high detection temperature (see Fig. 1D and (i)): $F_H - [F_L \div RV_F]$.

In considering embodiments described above, one of skill in the art would understand that the ratio of the detected signal ($FT_H$) at the relatively high detection temperature provided by signal-generating means to the detected signal ($FT_L$) at the relatively low detection temperature provided by signal-generating means is calculated and used as a reference value (*i.e.* $RV = (FT_H) \div (FT_L)$), thereby determining the presence or absence of target nucleic acid sequences in accordance with the present method. However, in appended claims protection is sought for the method defined in the claims, only.

According to an embodiment, the present disclosure further uses a threshold value for determining significance of the calculation results by the above-described equations. Depending on selected equations, different threshold value may be applied. According to an embodiment, the threshold value may be selected to mutually supplement with the reference value. However, the scope of the claims is characterise by the calculation as defined in the claims, only.

According to an embodiment, where signals are generated in a real-time manner associated with target amplification by PCR, the signals at each amplification cycle or some selected cycles are mathematically processed with the reference values and the calculation results are plotted against cycles and used for determination of the presence of the target nucleic acid sequence.

According to an embodiment, the single reaction vessel further comprises at least one additional set each of which contains additional two signal-generating means for detection of target nucleic acid sequences other than the two target nucleic acid sequences; wherein the signals generated by each set of two signal-generating means in the vessel are differentiated from each other and the signals are detected by different types of detectors, respectively, However, the scope of the claims is characterised by the use of only one type of detector which does not differentiate the signals. For example, where the two signal-generating means in the step (b) are labeled with FAM and the additional two signal-generating means are labeled with Quasar 570, the signals generated by FAM-labeled signal-generating means in the vessel are differentiated from the signals generated by Quasar 570-labeled signal-generating means and therefore two types of detectors are required to detect two different emission lights.

According to an embodiment of this invention, the two target nucleic acid sequences comprises a nucleotide variation and one of the two target nucleic acid sequences comprises one type of the nucleotide variation and the other comprises the other type of the nucleotide variation.

The term "nucleotide variation" used herein refers to any single or multiple nucleotide substitutions, deletions or insertions in a DNA sequence at a particular location among contiguous DNA segments that are otherwise similar in sequence. Such contiguous DNA segments include a gene or any other portion of a chromosome. These nucleotide variations may be mutant or polymorphic allele variations. For example, the nucleotide variation detected in the present invention includes SNP (single nucleotide polymorphism), mutation, deletion, insertion, substitution and translocation. Exemplified nucleotide variation includes numerous variations in a human genome (e.g., variations in the MTHFR (methylenetetrahydrofolate reductase) gene), variations involved in drug resistance of pathogens and tumorigenesis-causing variations. The term nucleotide variation used herein includes any variation at a particular location in a nucleic acid sequence. In other words, the term nucleotide variation includes a wild type and its any mutant type at a particular location in a nucleic acid sequence.

According to an embodiment of this invention, the nucleotide variation detected by the present invention is a SNP (single nucleotide polymorphism).

According to an embodiment of this invention, a homozygote composed of a first SNP allele is detected by using a first signal-generating means, a homozygote composed of a second SNP allele by using a second signal-generating means and a heterozygote composed of the first SNP allele and the second SNP allele by using the first signal-generating means and the second signal-generating means.

Under the performance principle underlying the present invention, the present method may be applied to detection of at least target nucleic acid sequence of three target nucleic acid sequences.

For instance, the three target nucleic acid sequence comprise a first target nucleic acid sequence, a second target nucleic acid sequence and a third target nucleic acid sequence. Among them, the first target nucleic acid sequence may be detected as follows:
Where the second target nucleic acid sequence and the third target nucleic acid sequence is collectively considered as a single target, a collective reference value for the single target may be obtained by incubating the second target nucleic acid sequence and the third target nucleic acid sequence with the second signal-generating means and the third signal-generating means. Alternatively, either the second reference value for the second target nucleic acid sequence or the third reference value for the third target nucleic acid sequence may be utilized as the collective reference value.

Then, the presence or absence of the first target nucleic acid sequence may be determined by using the collective reference value and signals at detected at the two detection temperatures.

Also, the presence or absence of each of the second target nucleic acid sequence and the third target nucleic acid sequence may be determined in accordance with the detection approach for the first target nucleic acid sequence.

**II. SNP Genotyping of a Nucleic Acid Sequence in a Sample Using Different Detection Temperatures and Reference Values**

In still another aspect of this invention, there is provided a method for SNP (single nucleotide polymorphism) genotyping of a nucleic acid sequence in a sample using different detection temperatures and reference values, as defined in the appended claims, comprising:

(a) providing (i) a first reference value for a homozygote composed of a first SNP allele which represents a relationship of change in signals provided at a relatively high detection temperature and a relatively low detection temperature by a first signal-generating means; (ii) a second reference value for a homozygote composed of a second SNP allele which represents a relationship of change in signals provided at a relatively high detection temperature and a relatively low detection temperature by a second signal-generating means; and (iii) a third reference value for a heterozygote composed of the first SNP allele and the second SNP allele which represents a relationship of change in signals provided at a relatively high detection temperature and a relatively low detection temperature by the first signal-generating means and the second signal-generating means; wherein the three reference values are different from each other;

(b) incubating the sample with the first signal-generating means and the second signal-generating means for the SNP alleles and detecting signals from the two signal-generating means at the relatively high detection temperature and the relatively low detection temperature; wherein the two signal-generating means generate signals at the relatively high detection temperature and at the relatively low detection temperature; wherein signals to be generated by the two signal-generating means are not differentiated by a single type of detector; and

(c) determining a SNP genotype by the reference values and a difference between the signals detected at the relatively high detection temperature and the relatively low detection temperature in the step (b).

Since the present invention follows in principle the first aspect of this invention described above, the common descriptions between them are omitted in order to avoid undue redundancy leading to the complexity of this specification. When referring to descriptions for the first aspect in order to describe this aspect, it should be noted that this aspect is, in part, different from the first aspect. Therefore, it would be understood to those skilled in the art that some descriptions for the first aspect may be directly applied to descriptions for this aspect and other descriptions with modifications may be applied to descriptions for this aspect, which is defined by the appended claims.

**Step (a): <u>Providing reference values</u>**

The first reference value for a homozygote composed of a first SNP allele, the second reference value for a homozygote composed of a second SNP allele and the third reference value for a heterozygote composed of the first SNP allele and the second SNP allele are provided.

The present invention has features in which reference values for the three genotypes are utilized.

Each reference value may be obtained by incubating the corresponding SNP type and signal-generating means, detecting signals at a relatively high detection temperature and a relatively low detection temperature, and then obtaining a difference between the signals detected at a relatively high detection temperature and a relatively low detection temperature.

According to an embodiment, the signal-generating means are designed such that the three reference values are different from each other.

According to an embodiment, (i) a first reference value for a homozygote composed of a first SNP allele is obtained by (i-1) incubating the homozygote composed of the first SNP allele with a first signal-generating means for detection of the first SNP allele, (i-2) detecting signals at a relatively high detection temperature and a relatively low detection temperature, and (i-3) then obtaining a difference between the signals detected at the relatively high detection temperature and the relatively low detection temperature, (ii) a second reference value for a homozygote composed of a second SNP allele is obtained by (ii-1) incubating the homozygote composed of the second SNP allele with a second signal-generating means for detection of the second SNP allele, (ii-2) detecting signals at the relatively high detection temperature and the relatively low detection temperature, and (ii-3) then obtaining a difference between the signals detected at the relatively high detection temperature and the relatively low detection temperature; and (iii) a third reference value for a heterozygote composed of the first SNP allele and the second SNP allele is obtained by (iii-1) incubating the heterozygote composed of the first SNP allele and the second SNP allele with the first signal-generating means for detection of the first SNP allele and the second signal-generating means for detection of the second SNP allele, (iii-2) detecting signals at the relatively high detection temperature and the relatively low detection temperature, and (iii-3) then obtaining a difference between the signals detected at the relatively high detection temperature and the relatively low detection temperature.

According to an embodiment, the signal-generating means are designed such that the reference value for the first SNP allele is different from reference value for the second SNP allele.

The nucleic acid sequence containing a SNP site may include a chromosome pair of human.

According to an embodiment, a difference between the signals detected in obtaining the reference values represents a relationship of change in signals provided at a relatively high detection temperature and a relatively low detection temperature.

According to the claimed embodiments, the difference between the signals detected in obtaining the reference values is ratio between the signals.

According to an embodiment of this invention, the difference between the signals obtained in obtaining the reference value has a certain range and the reference value is selected within the certain range or with referring to the certain range. According to an embodiment of this invention, the reference value may be selected with maximum or minimum value of the certain range or with referring to maximum or minimum value of the certain range.

According to an embodiment of this invention, the reference value may be obtained under reaction conditions sufficient to provide a saturated signal at the reaction completion. For example, in order to obtain a reference value for a heterozygote composed of both of the first SNP allele and the second SNP allele, the reaction conditions such as the content of each SNP allele are selected such that a saturated signal for each SNP allele is provided at the reaction completion. According to an embodiment of this invention, the ratio of the signals obtained in calculating the reference value has a certain range and the reference value is selected within the certain range or with referring to the certain range. According to an embodiment of this invention, the reference value may be selected with maximum or minimum value of the certain range or with referring to maximum or minimum value of the certain range.

### Step (b): Incubating samples with signal-generating means and signal detection

The sample comprising the nucleic acid sequence containing a SNP (single nucleotide polymorphism) site is incubated with the first signal-generating means and the second signal-generating means for SNP genotyping of a nucleic acid sequence and signals from the two signal-generating means are detected at the relatively high detection temperature and the relatively low detection temperature. The two signal-generating means generate signals at the relatively high detection temperature and the relatively low detection temperature and signals to be generated by the two signal-generating means are not differentiated by a single type of detector.

According to an embodiment of this invention, the step (b) is performed in a signal amplification process concomitantly with a nucleic acid amplification.

According to an embodiment of this invention, the step (b) is performed in a signal amplification process without a nucleic acid amplification.

### Step (c): Determining a SNP genotype

Finally, a SNP genotype is determined by the reference values and a ratio of the signals detected at the relatively high detection temperature and the relatively low detection temperature in the step (b).

The present invention allows for SNP genotyping only by the reference values of the corresponding SNP genotype and a ratio of the signals detected at the relatively high detection temperature and the relatively low detection temperature with no determining which SNP alleles are present in the sample.

The reason for such no requirement for determining the presence of the individual SNP allele is that there are three SNP genotypes and a heterozygote for SNP comprises the wild type allele and the mutant allele in 1:1 ratio. In addition, the reason is that the amount of nucleic acid molecules in sample incubation becomes easily adjustable.

According to an embodiment, the difference between the signals detected in the step (b) comprises a difference to be obtained by mathematically processing the signal detected at the relatively high detection temperature and the signal detected at the relatively low detection temperature.

According to the appended claims, the difference between the signals detected in the step (b) is obtained by calculating the ratio between the signals.

According to an embodiment of this invention, the homozygote sample containing the first SNP allele shows a difference as the ratio within a certain range, the heterozygote sample shows a difference as the ratio within another certain range and the homozygote sample containing the second SNP allele shows a difference as the ratio within the other certain range.

According to an embodiment, which is not claimed, the SNP genotype is determined by comparing the ration of the signals with the reference values. For example, where the first reference value for the homozygote composed of the first SNP allele is 1.0, the second reference value for the homozygote composed of the second SNP allele is 5.2, the third reference value for the heterozygote composed of the first SNP allele and the second SNP allele is 3.2, and the difference between the signals in the step (b) is substantially 1.0, the sample is determined to be the homozygote of the first SNP allele.

According to an embodiment, two cut-off values with considering the ranges of reference values for each genotype may be established and used for genotyping.

**IV. Kits for Detection of Target Nucleic Acid** Sequences, which are not claimed.

In further aspect of this disclosure, there is provided a kit for detecting at least one target nucleic acid sequences of two target nucleic acid sequences in a sample using different detection temperatures and reference values, comprising:

> (a) two signal-generating means for detection of the two target nucleic acid sequences; and
> (b) an instruction that describes the present method of the Aspect I titled as Detection of Two Target Nucleic Acid Sequences in a Sample Using Different Detection Temperatures and Reference Values.

In still further aspect of this disclosure, there is provided a kit for detecting at least one target nucleic acid sequences of two target nucleic acid sequences in a sample using different detection temperatures and reference values, comprising:

> (a) two signal-generating means for detection of at least one of the two target nucleic acid sequences; and
> (b) an instruction that describes the present method of the Aspect II titled as Detection of At Least One Target Nucleic Acid Sequence in a Sample Using Different Detection Temperatures and Reference Values.

In another aspect of this disclosure, there is provided a kit for SNP (single nucleotide polymorphism) genotyping of a nucleic acid sequence in a sample using different detection temperatures and reference values, comprising:

> (a) a signal-generating means for a first SNP allele;
> (b) a signal-generating means for a second SNP allele; and
> (c) an instruction that describes the present method of the Aspect III titled as SNP Genotyping of a Nucleic Acid Sequence in a Sample Using Different Detection Temperatures and Reference Values.

Since the kits of this disclosure, which are not claimed, are prepared to perform the present methods, the common descriptions between them are omitted in order to avoid undue redundancy leading to the complexity of this specification.

All of the present kits described hereinabove may optionally include the reagents required for performing target amplification PCR reactions (e.g., PCR reactions) such as buffers, DNA polymerase cofactors, and deoxyribonucleotide-5-triphosphates. Optionally, the kits may also include various polynucleotide molecules, reverse transcriptase, various buffers and reagents, and antibodies that inhibit DNA polymerase activity. The kits may also include reagents necessary for performing positive and negative control reactions. Optimal amounts of reagents to be used in a given reaction can be readily determined by the skilled artisan having the benefit of the current disclosure. The components of the kit may be present in separate containers, or multiple components may be present in a single container.

The instructions for describing or practicing the methods of the present invention may be recorded on a suitable recording medium. For example, the instructions may be printed on a substrate, such as paper and plastic. In other embodiments, the instructions may be present as an electronic storage data file present on a suitable computer readable storage medium such as CD-ROM and diskette. In yet other embodiments, the actual instructions may not be present in the kit, but means for obtaining the instructions from a remote source, *e.g.* via the internet, are provided. An example of this embodiment is a kit that includes a web address where the instructions can be viewed and/or from. which the instructions can be downloaded.

**V. Storage medium and Device for Detection of Target Nucleic Acid Sequences**

Since the storage medium, the device and the computer program of the prevent invention described hereinbelow are intended to perform the present methods in a computer, the common descriptions between them are omitted in order to avoid undue redundancy leading to the complexity of this specification.

In another aspect of this invention, there is provided a computer readable storage medium, as defined in the appended claims, containing instructions to configure a processor to perform a method for determining the presence of at least one target nucleic acid sequence of two target nucleic acid sequences comprising a first target nucleic acid sequence and a second target nucleic acid sequence in a sample using different detection temperatures and reference values, the method comprising:

(a) receiving signals in the sample generated from a first signal-generating means for the first target nucleic acid sequence and a second signal-generating means for the second target nucleic acid sequence at a relatively high detection temperature and a relatively low detection temperature; wherein the two signal-generating means generate signals at the relatively high detection temperature and the relatively low detection temperature; wherein signals to be generated by the two signal-generating means are not differentiated by a single type of detector; and

(b) determining the presence of at least one target nucleic acid sequence by the signals received in the step (a), and a first reference value for the first target nucleic acid sequence and/or a second reference value for the second target nucleic acid sequence; wherein the first reference value represents a relationship of change in signals provided at a relatively high detection temperature and a relatively low detection temperature by a first signal-generating means, and the second reference value represents a relationship of change in signals provided at a relatively high detection temperature and a relatively low detection temperature by a second signal-generating means; wherein the first reference value is different from the second reference value.

According to an embodiment of the present invention, the first reference value and/or the second reference value is stored in the computer readable storage medium. According to an embodiment of the present invention, the computer readable storage medium contains instructions to input the first reference value and/or the second reference value in performing the method. According to an embodiment of the present invention, the computer readable storage medium further contains instructions to configure a processor to perform a method for obtaining the first reference value and/or the second reference value.

In still another aspect of this invention, there is provided a computer program to be stored on a computer readable storage medium to configure a processor to perform a method for determining the presence of at least one target nucleic acid sequence of two target nucleic acid sequences comprising a first target nucleic acid sequence and a second target nucleic acid sequence in a sample using different detection temperatures and reference values, the method comprising:

(a) receiving signals in the sample generated from a first signal-generating means for the first target nucleic acid sequence and a second signal-generating means for the second target nucleic acid sequence at a relatively high detection temperature and a relatively low detection temperature; wherein the two signal-generating means generate signals at the relatively high detection temperature and the relatively low detection temperature; wherein signals to be generated by the two signal-generating means are not differentiated by a single type of detector; and

(b) determining the presence of at least one target nucleic acid sequence by the signals received in the step (a), and a first reference value for the first target nucleic acid sequence and/or a second reference value for the second target nucleic acid sequence; wherein the first reference value represents a relationship of change in signals provided at a relatively high detection temperature and a relatively low detection temperature by a first signal-generating means, and the second reference value represents a relationship of change in signals provided at a relatively high detection temperature and a relatively low detection temperature by asecond signal-generating means; wherein the first reference value is different from the second reference value, as defined in the appended claims.

According to an embodiment of the present invention, the computer program contains the first reference value and/or the second reference value. According to an embodiment of the present invention, the computer program contains instructions to input the first reference value and/or the second reference value in performing the method. According to an embodiment of the present invention, the computer program further contains instructions to configure a processor to perform a method for obtaining the first reference value and/or the second reference value.

The program instructions are operative, when preformed by the processor, to cause the processor to perform the present method described above. The program instructions may comprise an instruction to receive the first signal and the second signal, and an instruction to determine the presence of the two target nucleic acid sequences by using the signals received.

The present method described above is implemented in a processor, such as a processor in a stand-alone computer, a network attached computer or a data acquisition device such as a real-time PCR machine.

The types of the computer readable storage medium include various storage medium such as CD-R, CD-ROM, DVD, flash memory, floppy disk, hard drive, portable HDD, USB, magnetic tape, MINIDISC, nonvolatile memory card, EEPROM, optical disk, optical storage medium, RAM, ROM, system memory and web server.

The data *(e.g.,* intensity, amplification cycle number and detection temperature) associated with the signals may be received through several mechanisms. For example, the data may be acquired by a processor resident in a PCR data acquiring device. The data may be provided to the processor in real time as the data is being collected, or it may be stored in a memory unit or buffer and provided to the processor after the experiment has been completed. Similarly, the data set may be provided to a separate system such as a desktop computer system via a network connection *(e.g.,* LAN, VPN, intranet and Internet) or direct connection *(e.g.,* USB or other direct wired or wireless connection) to the acquiring device, or provided on a portable medium such as a CD, DVD, floppy disk, portable HDD or the like to a stand-alone computer system. Similarly, the data set may be provided to a server system via a network connection (e.g., LAN, VPN, intranet, Internet and wireless communication network) to a client such as a notebook or a desktop computer system. After the data has been received or acquired, the data analysis process proceeds to give a processed signal obtained from a difference between the signals for determination of the presence of target nucleic acid sequences when the signal is detected at the relatively high detection temperature. The processor processes the received data associated with the signals to give the processed signal reflecting the difference between the signals in the two detection temperatures. For example, the processor processes the received data to obtain a ratio of the signal detected at the relatively low detection temperature to the signal detected at the relatively high detection temperature.

The instructions to configure the processor to perform the present invention may be included in a logic system. The instructions may be downloaded and stored in a memory module (e.g., hard drive or other memory such as a local or attached RAM or ROM), although the instructions can be provided on any software storage medium such as a portable HDD, USB, floppy disk, CD and DVD. A computer code for implementing the present invention may be implemented in a variety of coding languages such as C, C++, Java, Visual Basic, VBScript, JavaScript, Perl and XML. In addition, a variety of languages and protocols may be used in external and internal storage and transmission of data and commands according to the present invention.

In further aspect of this invention, there is provided a device, as defined in the appended claims, for determining the presence of at least one target nucleic acid sequence of two target nucleic acid sequences comprising a first target nucleic acid sequence and a second target nucleic acid sequence in a sample using different detection temperatures and reference values, comprising (a) a computer processor and (b) the computer readable storage medium described above coupled to the computer processor.

According to an embodiment, the device further comprises a reaction vessel to accommodate the sample and signal-generating means, a temperature controlling means to control temperatures of the reaction vessel and/or a single type detector to detect signals to be generated by the signal-generating means.

According to an embodiment, the computer processor permits not only the single type of detector to detect signals generated by the signal-generating means at a relatively high detection temperature and a relatively low detection temperature but also to calculate a difference as a ratio of the signals detected at the relatively high detection temperature and the relatively low detection temperature. The processor may be prepared in such a manner that a single processor. can do two performances: direction of detection at two detection temperatures and calculation of the ratio. Alternatively, the processor unit may be prepared in such a manner that two processors do two performances, respectively.

The first essential feature of the device carries the processor to permit the device to detect signals to be generated at the two detection temperatures. According to an embodiment, where the signal is generated along with amplification of the target nucleic acid sequence, the device comprises a processor to permit the device to detect signals to be generated at the two detection temperatures at each amplification cycle.

The second essential feature of the device is to carry the processor to process the signal detected at the two detection temperatures, to obtain the difference between the signals. According to an embodiment, the difference between the signals is expressed as numeric values by a mathematical processing.

According to an embodiment, the processor may be embodied by installing software into conventional devices for detection of target nucleic acid sequences (e.g. real-time PCR device). According to an embodiment, the device comprises a processor to permit the device to detect signals at two detection temperatures and to mathematically process two detection results.

In another aspect of this invention, there is provided a computer readable storage medium, as defined in the appended claims, containing instructions to configure a processor to perform a method for SNP (single nucleotide polymorphism) genotyping of a nucleic acid sequence in a sample using different detection temperatures and reference values, the method comprising:

    (a) receiving signals in the sample generated from a first signal-generating means and a second signal-generating means for SNP alleles at a relatively high detection temperature and a relatively low detection temperature; wherein the two signal-generating means generate signals at the relatively high detection temperature and at the relatively low detection temperature; wherein signals to be generated by the two signal-generating means are not differentiated by a single type of detector; and
    (b) determining a SNP genotype by a ratio of the signals received in the step (a), and a first reference value for a homozygote composed of a first SNP allele, a second reference value for a homozygote composed of a second SNP allele and a third reference value for a heterozygote composed of the first SNP allele and the second SNP allele; wherein the first reference value represents a relationship of change in signals provided at a relatively high detection temperature and a relatively low detection temperature by a first signal-generating means, the second reference value represents a relationship of change in signals provided at a relatively high detection temperature and a relatively low detection temperature by a second signal-generating means, and the third reference value represents a relationship of change in signals provided at a relatively high detection temperature and a relatively low detection temperature by the first signal-generating means and the second signal-generating means; wherein the three reference values are different from each other.

According to an embodiment of the present invention, the first reference value and/or the second reference value and/or the third reference value is stored in the computer readable storage medium. According to an embodiment of the present invention, the computer readable storage medium contains instructions to input the first reference value and/or the second reference value and/or the third reference value in performing the method. According to an embodiment of the present invention, the computer readable storage medium further contains instructions to configure a processor to perform a method for obtaining the first reference value and/or the second reference value and/or the third reference value.

In still another aspect of this invention, there is provided a computer program to be stored on a computer readable storage medium to configure a processor to perform a method for SNP (single nucleotide polymorphism) genotyping of a nucleic acid sequence in a sample using different detection temperatures and reference values, the method comprising:

(a) receiving signals in the sample generated from a first signal-generating means and a second signal-generating means for SNP alleles at a relatively high detection temperature and a relatively low detection temperature; wherein the two signal-generating means generate signals at the relatively high detection temperature and at the relatively low detection temperature; wherein signals to be generated by the two signal-generating means are not differentiated by a single type of detector; and

(b) determining a SNP genotype by a ratio of the signals received in the step (a), and a first reference value for a homozygote composed of a first SNP allele, a second reference value for a homozygote composed of a second SNP allele and a third reference value for a heterozygote composed of the first SNP allele and the second SNP allele; wherein the first reference value represents a relationship of change in signals provided at a relatively high detection temperature and a relatively low detection temperature by a first signal-generating means, the second reference value represents a relationship of change in signals provided at a relatively high detection temperature and a relatively low detection temperature by a second signal-generating means, and the third reference value represents a relationship of change in signals provided at a relatively high detection temperature and a relatively low detection temperature by the first signal-generating means and the second signal-generating means; wherein the three reference values are different from each other.

According to an embodiment of the present invention, the computer program contains the first reference value and/or the second reference value and/or the third reference value. According to an embodiment of the present invention, the computer program contains instructions to input the first reference value and/or the second reference value and/or the third reference value in performing the method. According to an embodiment of the present invention, the computer program further contains instructions to configure a processor to perform a method for obtaining the first reference value and/or the second reference value and/or the third reference value.

In further aspect of this invention, there is provided a device for SNP (single nucleotide polymorphism) genotyping of a nucleic acid sequence in a sample using different detection temperatures and reference values, comprising (a) a computer processor and (b) the computer readable storage medium described above coupled to the computer processor.

The features and advantages of this invention will be summarized as follows:

(a) The present invention employing different detection temperatures enables to detect a plurality of target nucleic acid sequences in conventional real-time manners even with a single type of label in a single reaction vessel. The conventional technologies detect a plurality of target nucleic acid sequences by a melting analysis after target amplification. As opposed to this, the present invention does not require a melting analysis after target amplification, such that the time for analysis is greatly reduced.

(b) Even when signals for two target nucleic acid sequence are generated at two detection temperatures, the present invention can detect each target nucleic acid sequence. Such advantage makes it possible to use signal-generating means to generate signals by cleavage for each target nucleic acid sequence.

(c) In the present invention using different detection temperatures, for each of target nucleic acid sequences, the use of a signal-generating means to provide a signal by a duplex formed in a dependent manner on cleavage of a mediation oligonucleotide specifically hybridized with a target nucleic acid sequence (e.g., PTOCE-based methods) can induce the unexpected results. First, methods using the mediation oligonucleotide such as the PTOCE-based methods can readily adjust $T_m$ value of duplex formed to ensure convenient selection of detection temperatures. By such features, it becomes more conveniently adjustable to have desired reference values (or difference in reference values). Furthermore, in the methods using the mediation oligonucleotide such as the PTOCE-based methods, a duplex having a certain $T_m$ value can be formed because the duplex has a sequence irrespective of a target nucleic acid sequence. Unlikely, in methods using probes to be directly hybridized with a target nucleic acid sequence, because at least one strand of a duplex formed comprises a sequence complementary to a target nucleic acid sequence, a duplex having $T_m$ value not intended may be formed when a variation on the target nucleic acid sequence is present.

The present invention will now be described in further detail by examples. It would be obvious to those skilled in the art that these examples are intended to be more concretely illustrative and the scope of the present invention as set forth in the appended claims is not limited to or by the examples.

## EXAMPLES

### EXAMPLE 1: Multiple Target Detection by TaqMan real-time PCR Using Different Detection Temperatures and Reference Values

We examined whether two target nucleic acids in samples can be detected in a single reaction vessel by using a single detection channel. The following detection processes were performed using different detection temperatures and reference values and TaqMan real-time PCR was applied as signal generating means.

*Taq* DNA polymerase having a 5' nuclease activity was used for the extension of upstream primers and downstream primers and the cleavage of TaqMan probes. The genomic DNA of *Neisseria gonorrhoeae* (NG) and genomic DNA of *Chlamydia trachomatis* (CT) were used as target nucleic acid sequences. Four types of samples (NG, CT, NG+CT and no target control) were prepared and analyzed.

TaqMan real-time PCR was employed to detect NG and CT. Where a target nucleic acid is present, a TaqMan probe is cleaved and a labeled fragment is released. An amplification curve can be obtained by measuring signal from the labeled fragment.

A TaqMan probe for NG was labeled with a fluorescent reporter molecule (Quasar 670) at its 5'-end and a quencher molecule at its 3'-end (SEQ ID NO: 3) and a TaqMan probe for CT with a fluorescent reporter molecule (Quasar 670) at its 5'-end and a quencher molecule in its inner part (BHQ-2) (SEQ ID NO: 6).

In this Example, even though the signals from the TaqMan probes are not distinguishable from each other, plotting methods using reference values for each target sequence and signals from two detection temperatures (72°C and 60°C) provide amplification curves indicating the presence of each target nucleic acid sequence.

Calculation equations for reference values and plotting equations to obtain amplification curves for each target nucleic acid sequence were as follows:

(1) Reference value (RV) of NG or CT
RFU at 60°C ÷ RFU at 72°C at the end-point
(2) Plotting equations for NG target:

(i) RFU at 72°C - (RFU at 60°C ÷ RV of CT target)
or (ii) RFU at 60°C - (RFU at 72°C X RV of CT target)

(3) Plotting equations for CT target:

(i) RFU at 72°C - (RFU at 60°C ÷ RV of NG target)
or (ii) RFU at 60°C - (RFU at 72°C X RV of NG target)

In the equations, the RFU (relative fluorescent unit) values are those measured at each cycle of real-time PCR and RV denotes a reference value.

The sequences of upstream primers, downstream primers and probes used in this Example are:

NG-F 5'-TACGCCTGCTACTTTCACGCTIIIIIGTAATCAGATG-3' (SEQ ID NO: 1)
NG-R 5'-CAATGGATCGGTATCACTCGCIIIIICGAGCAAGAAC-3' (SEQ ID NO: 2)
NG-P 5'-[Quasar 670]TGCCCCTCATTGGCGTGTTTCG[BHQ-2]-3' (SEQ ID NO: 3)

CT-F1 5'-TCCGAATGGATAAAGCGTGACIIIIIATGAACTCAC-3' (SEQ ID NO: 4)
CT-R1 5'-AACAATGAATCCTGAGCAAAGGIIIIICGTTAGAGTC-3' (SEQ ID NO: 5)
CT-P 5'-[Quasar 670]CATTGTAAAGA[T(BHQ-2)]ATGGTCTGCTTCGACCG[C3 spacer]-3' (SEQ ID NO: 6)
(I: Deoxyinosine)

The real-time PCR was conducted in the final volume of 20 μl containing a target nucleic acid (1 pg of NG genomic DNA, 10 pg of CT genomic DNA or a mixture of 1 pg of NG genomic DNA and 10 pg of CT genomic DNA), 5 pmole of upstream primer (SEQ ID NO: 1) and 10 pmole of downstream primer (SEQ ID NO: 2) for NG target amplification, 1.5 pmole of TaqMan probe (SEQ ID NO: 3), 5 pmole of upstream primer (SEQ ID NO: 4) and 10 pmole of downstream primer (SEQ ID NO: 5) for CT target amplification, 3 pmole of TaqMan probe (SEQ ID NO: 6), and 5 μl of 4X Master Mix [final, 200 μM dNTPs, 2 mM MgCl$_2$, 2 U of *Taq* DNA polymerase]. The tubes containing the reaction mixture were placed in the real-time thermocycler (CFX96, Bio-Rad) for 5 min at 50°C, denatured for 15 min at 95°C and subjected to 50 cycles of 30 sec at 95°C, 60 sec at 60°C, 30 sec at 72°C. The detection of signals was performed at 60°C and 72°C at each cycle.

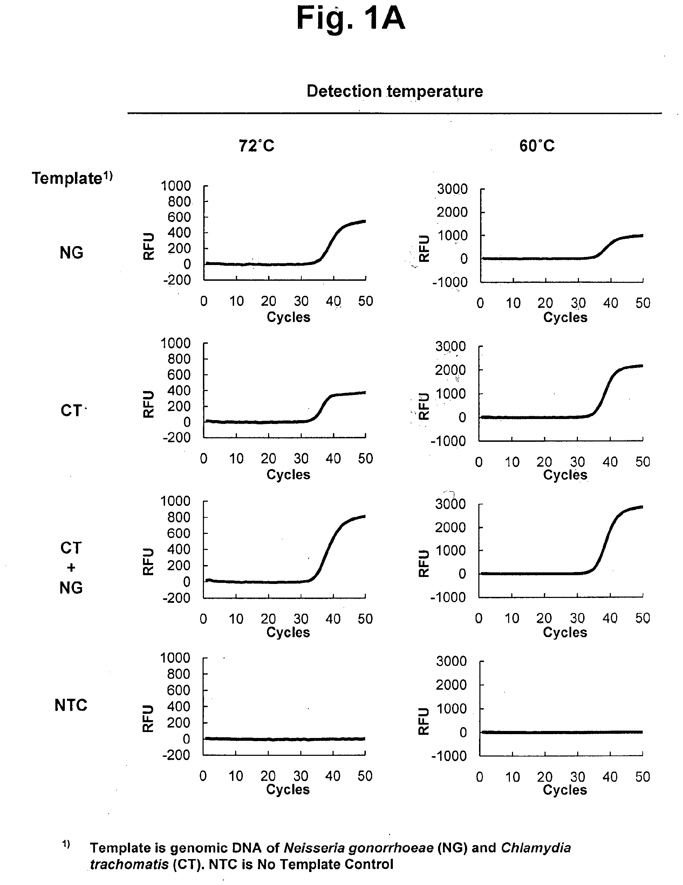

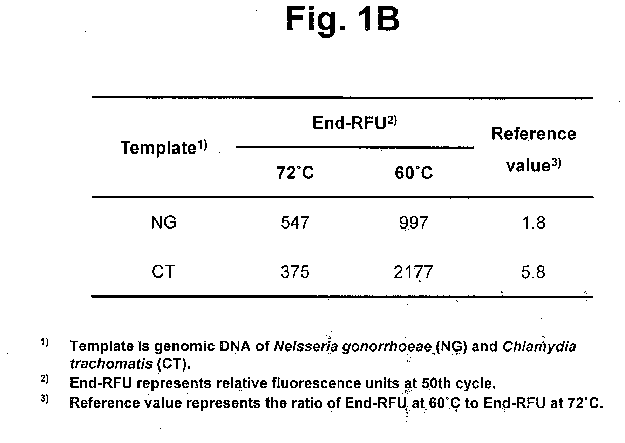

As shown in Fig. 1A, signals were detected both at 60°C and 72°C in the presence of NG, CT, or NG + CT. No signal was detected in the absence of the target nucleic acids. Reference values for each target sequences were calculated using the signals of NG only sample or CT only sample. As shown in Fig. 1B, reference values for NG and CT target were 1.8 and 5.8, respectively.

Then, to identify target sequences, the corresponding reference value and RFU at 72°C and 60°C were applied to plotting equations ((i) or (ii) equations in Fig. 1C and Fig. 1D) and amplification curves of each target sequences were obtained. Proper thresholds were selected referring to the result of NG only sample and CT only sample to ensure the significance of the obtained amplification curves.

As shown in Fig. 1C and Fig. 1D, the amplification curves derived from the plotting methods can identify the presence or absence of NG or CT in each sample.

Therefore, it can be appreciated that two target nucleic acids can be detected in a single reaction vessel even using a single detection channel by TaqMan real-time PCR using different detection temperatures and reference values.

### Example 2: Multiple target detection by PTOCE real-time PCR comprising Using Different Detection Temperatures and Reference Values

We examined whether two target nucleic acids in samples can be detected in a single reaction vessel by using a single detection channel. The following detection processes were performed using different detection temperatures and reference values and PTOCE real-time PCR was applied as signal generating means.

*Taq* DNA polymerase having a 5' nuclease activity was used for the extension of upstream primers and downstream primers, the cleavage of PTO, and the extension of PTO fragment. Genomic DNA of *Neisseria gonorrhoeae* (NG) and genomic DNA of *Chlamydia trachomatis* (CT) were used as target nucleic acid sequences. Four types of samples (NG, CT, NG+CT and no template control) were prepared and analyzed.

PTOCE real-time PCR was used to detect CT and NG. If a target is present, a PTO is cleaved and a PTO fragment is produced. The PTO fragment is annealed to the capturing portion of the CTO, extended on the templating portion of the CTO and forms an extended duplex with CTO (Duplexed CTO). The formation of the extended duplex provides a signal and an amplification curve can be obtained by measuring the signal at the extended duplex-forming temperature.

The PTO and CTO are blocked with a carbon spacer at their 3'-ends to prohibit their extension. The CTO is labeled with a quencher molecule (BHQ-2) and a fluorescent reporter molecule (CAL Fluor Red 610) in its templating portion (SEQ ID NOs: 8 and 12).

In this Example, 67.8°C and 60°C were selected as signal detection temperatures. The extended duplex produced depending on the presence of the target nucleic acid sequence has a controllable Tm value adjusted by their sequence and length. In this Example, the sequences and lengths of the extended duplexes for the NG and the CT are designed to provide a signal both, at 67.8°C and 60°C. Even though the signals from the signal-generating means are not distinguishable from each other, plotting methods using reference values for each target and signals from two detection temperatures (67.8°C and 60°C) provide amplification curves indicating the presence of each target nucleic acid sequence.

Calculation equations for reference values and plotting equations to obtain amplification curves for each target nucleic acid sequence were as follows:

(1) Reference value (RV) of NG or CT
RFU at 60°C ÷ RFU at 67.8°C at the end-point
(2) Plotting equations for NG target:

(i) RFU at 67.8°C - (RFU at 60°C ÷ RV of CT target)

or (ii) RFU at 60°C - (RFU at 67.8°C X RV of CT target)

(3) Plotting equations for CT target:

(i) RFU at 67.8°C - (RFU at 60°C ÷ RV of NG target)
or (ii) RFU at 60°C - (RFU at 67.8°C X RV of NG target)

In the equations, the RFU (relative fluorescent unit) values are those measured at each cycle of real-time PCR and RV denotes a reference value.

The sequences of upstream primer, downstream primer, PTO, and CTO used in this Example are:

NG-F 5'-TACGCCTGCTACTTTCACGCTIIIIIGTAATCAGATG-3' (SEQ ID NO: 1)
NG-R 5'-CAATGGATCGGTATCACTCGCIIIIICGAGCAAGAAC-3' (SEQ ID NO: 2)
NG-PTO 5'-<u>GTACGCGATACGGG</u>CCCCTCATTGGCGTGTTTCG[C3 spacer]-3' (SEQ ID NO: 7)
NG-CTO 5'- [BHQ-2]TTTTTTTTTTTTTTTTTTTTG[T(Cal Fluor Red 610)]ACTGCCCGTATCGCGTAC[C3 spacer]-3' (SEQ ID NO: 8)
CT-F2 5'-GAGTTTTAAAATGGGAAATICTGGTIIIIITTTGTATAAC-3' (SEQ ID NO: 9)
CT-R2 5'-CCAATTGTAATAGAAGCATTGGTTGIIIIITTATTGGAGA-3' (SEQ ID NO: 10)
CT-PTO 5'-<u>GATTACGCGACCG</u>CATCAGAAGCTGTCATTTTGGCTGCG[C3 spacer]-3' (SEQ ID NO: 11)
CT-CTO 5'-[BHQ-2]GCGCTGGATACCCTGGACGA[T(Cal Fluor Red 610)]ATGTGCGGTCGCGTAATC[C3 spacer]-3' (SEQ ID NO: 12)
(I: Deoxyinosine)

(Underlined letters indicate the 5'-tagging portion of PTO)

The real-time PCR was conducted in the final volume of 20 μl containing a target nucleic acid (10 pg of NG genomic DNA, 10 pg of CT genomic DNA or a mixture of 10 pg of NG genomic DNA and 10 pg of CT genomic DNA), 5 pmole of upstream primer (SEQ ID NO: 1) and 5 pmole of downstream primer (SEQ ID NO: 2) for NG target amplification, 3 pmole of PTO (SEQ ID NO: 7), 1 pmole of CTO (SEQ ID NO: 8), 5 pmole of upstream primer (SEQ ID NO: 9) and 5 pmole of downstream primer (SEQ ID NO: 10) for CT target amplification, 3 pmole of PTO (SEQ ID NO: 11), 1 pmole of CTO (SEQ ID NO: 12), and 5 μl of 4X Master Mix [final, 200 uM dNTPs, 2 mM $MgCl_2$, 2 U of *Taq* DNA polymerase]. The tubes containing the reaction mixture were placed in the real-time thermocycler (CFX96, Bio-Rad) for 5 min at 50°C, denatured for 15 min at 95°C and subjected to 50 cycles of 30 sec at 95°C, 60 sec at 60°C, 30 sec at 72°C, 5 sec at 67.8°C. Detection of a signal was performed at 60°C and 67.8°C of each cycle.

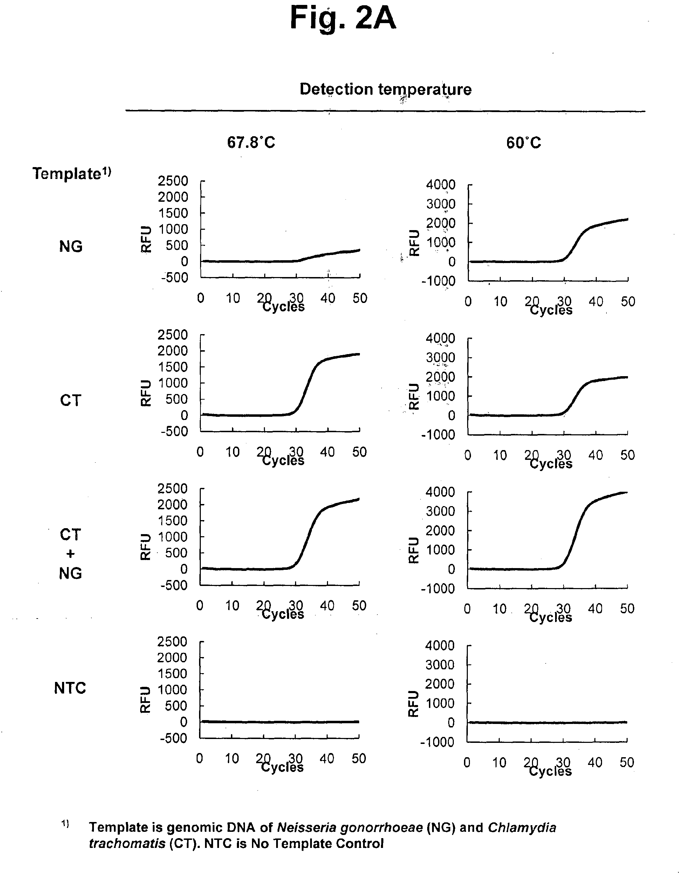

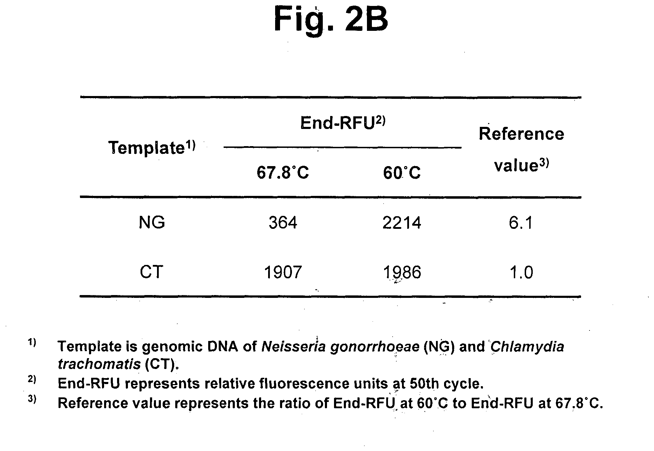

As shown in Fig. 2A, signals were detected both at 60°C and 67.8°C in the presence of NG, CT, or NG + CT. No signal was detected in the absence of the target nucleic acids. Reference values of each target were calculated using the signals of NG only sample or CT only sample. As shown in Fig. 2B, reference values for NG and CT target were 6.1 and 1.0, respectively.

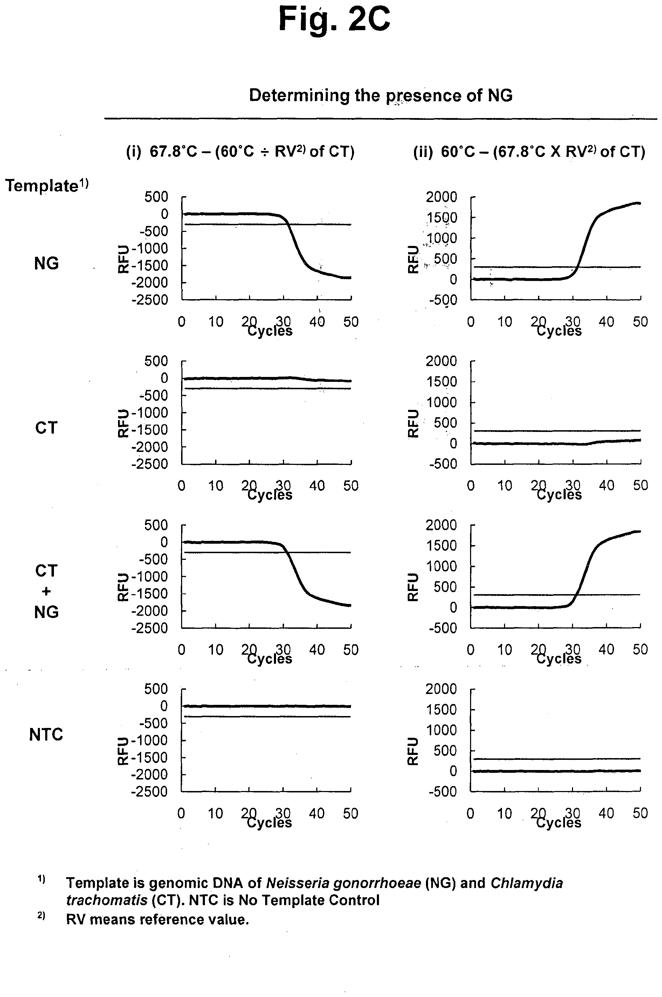

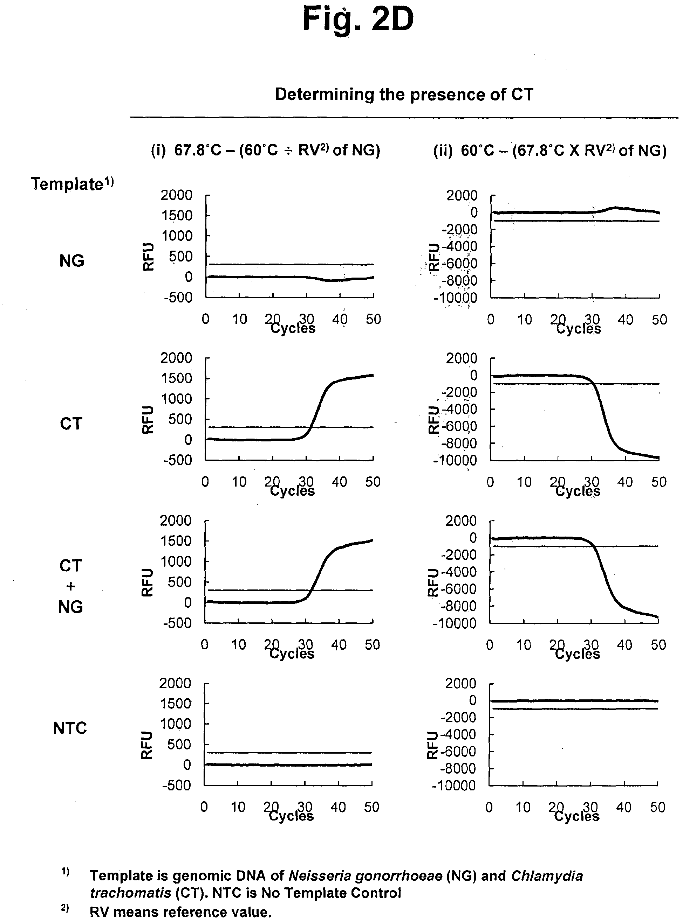

Then, to identify each sample, the corresponding reference value and RFU at 67.8°C and 60°C were applied to plotting equations ((i) or (ii) equations in Fig. 2C and Fig. 2D) and amplification curves of each target sequences were obtained. Proper thresholds were selected referring to the result of NG only sample and CT only sample to ensure the significance of the obtained amplification curves.

As shown in Fig. 2C and Fig. 2D, the amplification curves derived from the plotting methods can identify the presence or absence of NG or CT in each sample.

Therefore, two target nucleic acids can be detected in a single reaction vessel by using a single detection channel by PTOCE real-time PCR comprising signal detection at different temperatures.

Therefore, it can be appreciated that two target nucleic acids can be detected in a single reaction vessel using a single detection channel by PTOCE real-time PCR using different detection temperatures and reference values.

**EXAMPLE 3: SNP Genotyping Using Different Detection Temperatures and Reference Values**

We examined whether the present method can be applied to SNP genotyping in a single reaction vessel using a single detection channel. PTOCE real-time PCR was applied as signal generating means.

*Taq* DNA polymerase having a 5' nuclease activity was used for the extension of upstream primer and downstream primer, the cleavage of PTO, and the extension of PTO fragment. Wild (C) homozygote, mutant type (T) homozygote, and heterozygote of MTHFR (C677T) human genomic DNA were used as target nucleic acid sequences.

PTOCE real-time PCR was used to detect the wild (C) allele and mutant type (T) allele of the MTHFR (C677T) human genomic DNA. If a target allele is present, a PTO is cleaved and a PTO fragment is produced. The PTO fragment is annealed to the capturing portion of the CTO, extended on the templating portion of the CTO and forms an extended duplex with CTO (Duplexed CTO). The formation of the extended duplex provides a signal and an amplification curve can be obtained by measuring the signal at the extended duplex-forming temperature.

The PTO and CTO were blocked with a carbon spacer at their 3'-ends to prohibit their extension. The CTO for the wild (C) allele or the mutant type (T) allele was labeled with a quencher molecule (BHQ-2) at its 5'-end and a fluorescent reporter molecule (CAL Fluor Red 610) in its templating portion (SEQ ID NOs: 16 and 18).

In this Example, 64°C and 60°C were selected as signal detection temperatures. The extended duplex produced depending on the presence of the wild (C) allele or the mutant type (T) allele has a controllable $T_m$ value adjusted by their sequence and length. In this Example, the sequences and lengths of the extended duplex for the wild (C) allele and the mutant type (T) allele were designed to provide signal both at 64°C and 60°C. Reaction conditions comprising the extended duplexes and the two detection temperatures were designed and selected such that reference value of the wild (C) allele is different from that of the mutant type (T) allele.

The genotype of an unknown sample comprising MTHFR (C677T) gene can be determined by comparing the difference between the signals detected at the two detection temperatures with the reference values for the three genotypes.

In order to identify SNP genotype of the MTHFR (C677T) gene, reference values for each genotype were calculated. Because the reference values for each genotype are distinguishable, we may utilize two cut-off values to divide the range of reference values for each genotype. Reference values for each genotype used in this Example were calculated as follows:

Reference values for wild homozygote, mutant homozygote and heterozygote
RFU at 60°C ÷ RFU at 64°C at the end-point

In the equation, the RFU (relative fluorescent unit) values are those measured at the end-point of real-time PCR.

The sequences of upstream primer, downstream primer, PTO, and CTO used in this Example are:

M677-F 5'-CCACCCCGAAGCAGGGAIIIIIGAGGCTGACC-3' (SEQ ID NO: 13)
M677-R 5'-CAAGTGATGCCCATGTCGGIIIIIGCCTTCACAA-3' (SEQ ID NO: 14)
M677-W-PTO 5'-<u>GGTCCCGACGTTA</u>GCTCCCGCAGACACCTTCTCCTTC[C3 spacer]-3' (SEQ ID NO: 15)
M677-W-CTO 5'-[BHQ-2]CCTCGGTGCCACGCCATCGG[T(CAL Fluor Red 610)]TCTTCTAACGTCGGGACC[C3 spacer]-3' (SEQ ID NO: 16)
M677-M-PTO 5'-<u>ACGTCGATTCGC</u>ACTCCCGCAGACACCTTCTCCTTCAA[C3 spacer]-3' (SEQ ID NO: 17)
M677-M-CTO 5'-[BHQ-2]TTTTTTTTTTTTTTTTTTTTT[T(CAL Fluor Red 610)]ATTCTGCGAATCGACGT[C3 spacer]-3' (SEQ ID NO:18)
(I: Deoxyinosine)

(Underlined letters indicate the 5'-tagging portion of PTO)

The real-time PCR was conducted in the final volume of 20 μl containing a target nucleic acid (10 ng of wild (C) homozygous MTHFR (C677T) human genomic DNA, 10 ng of mutant (T) homozygous MTHFR (C677T) human genomic DNA, or 10 ng of heterozygous MTHFR (C677T) human genomic DNA), 5 pmole of upstream primer (SEQ ID NO: 13) and 5 pmole of downstream primer (SEQ ID NO: 14), 3 pmole of each PTO (SEQ ID NOs: 15 and 17), 1 pmole of each CTO (SEQ ID NOs: 16 and 18), and 5 μl of 4X Master Mix [final, 200 μM dNTPs, 2 mM MgCl$_2$, 2 U of *Taq* DNA polymerase]. The tubes containing the reaction mixture were placed in the real-time thermocycler (CFX96, Bio-Rad) for 5 min at 50°C, denatured for 15 min at 95°C and subjected to 50 cycles of 30 sec at 95°C, 60 sec at 60°C, 30 sec at 72°C, 5 sec at 64°C. Detection of a signal was performed at 60°C and 64°C of each cycle.

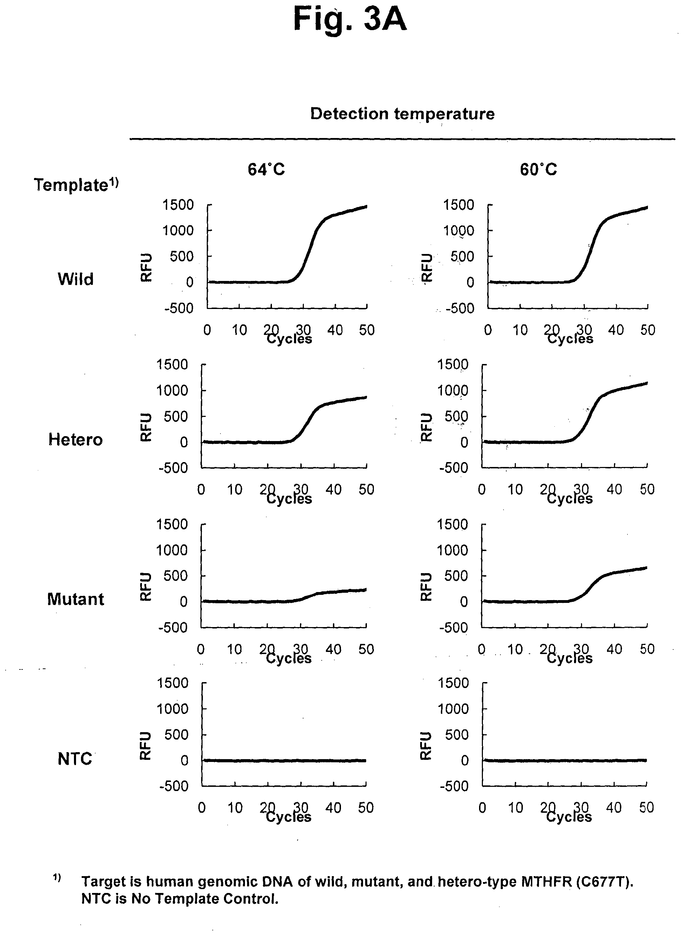

As shown in Fig. 3A, the fluorescence signals were detected both at 60°C and 64°C in the presence of the wild (C) homozygote, the mutant (T) homozygote, or the heterozygote. No signal was detected in the absence of the target nucleic acids. Reference values for three genotypes were calculated and confirmed that each genotype can be distinguished by cut-off values.

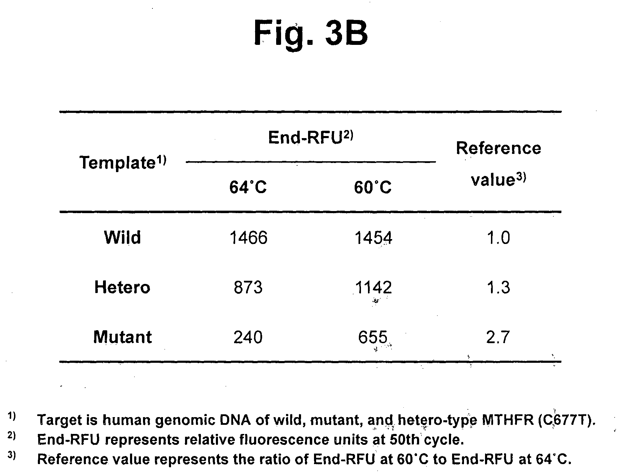

As shown in Fig. 3B, reference values for the wild (C) homozygote, the heterozygote, and the mutant (T) homozygote were 1.0, 1.3, and 2.7, respectively. Reference values of each genotype confirmed that each genotype can be distinguished.

These results indicate that the present method can be applied to SNP genotyping in a single reaction vessel by using a single detection channel. Interestingly, even though signals from alleles are not distinguishable from each other, the present method enables to accurately perform SNP genotyping even using a single detection channel.

Having described a preferred embodiment of the present invention, it is to be understood that variants and modifications thereof falling within Adaptation of description to the scope of the claims by ED. the invention may become apparent to those skilled in this art, and the scope of this invention is to be determined by appended claims.

**Claims**

1. A method for detecting at least one target nucleic acid sequences of two target nucleic acid sequences comprising a first target nucleic acid sequence and a second target nucleic acid sequence in a sample using different detection temperatures and reference values, comprising:

   (a) providing (i) a first reference value for the first target nucleic acid sequence which represents a ratio between signals provided at a relatively high detection temperature and a relatively low detection temperature by a first signal-generating means and (ii) a second reference value for the second target nucleic acid sequence which represents a ratio between signals provided at the relatively high detection temperature and the relatively low detection temperature by a second signal-generating means; wherein the first reference value is different from the second reference value; wherein the first reference value is obtained by (i) incubating the first target nucleic acid sequence with the first signal-generating means for detection of the first target nucleic acid sequence, (ii) detecting signals at the relatively high detection temperature and the relatively low detection temperature, and (iii) then obtaining a ratio between the signals detected at the relatively high detection temperature and the relatively low detection temperature; wherein the second reference value is obtained by (i) incubating the second target nucleic acid sequence with the second signal-generating means for detection of the second target nucleic acid sequence, (ii) detecting signals at the relatively high detection temperature and the relatively low detection temperature, and (iii) then obtaining a ratio between the signals detected at the relatively high detection temperature and the relatively low detection temperature;
   (b) incubating the sample with the first signal-generating means and the second signal-generating means for detection of the two target nucleic acid sequences and detecting signals from the two signal-generating means at the relatively high detection temperature and the relatively low detection temperature; wherein the two signal-generating means generate signals at the relatively high detection temperature and the relatively low detection temperature; wherein signals to be generated by the two signal-generating means are not differentiated by a single type of detector; and wherein the first signal-generating means comprises an oligonucleotide hybridized with the first target nucleic acid sequence and the second signal-generating means comprises an oligonucleotide hybridized with the second target nucleic acid sequence; wherein the relatively low detection temperature is a temperature at which the first signal-generating means does not generates signals indicating the presence of the second target nucleic acid sequence and the second signal-generating means does not generates signals indicating the presence of the first target nucleic acid sequence; wherein the incubating and detecting are performed by a real-time amplification reaction, not by melting analysis; and
   (c) determining the presence of the first target nucleic acid sequence by

   (i) [(the signal intensity at the relatively low detection temperature) - (the signal intensity at the relatively high detection temperature) * (the second reference value)];
   (ii) [(the signal intensity at the relatively low detection temperature) - (the signal intensity at the relatively high detection temperature) / (the second reference value)];
   (iii) [(the signal intensity at the relatively high detection temperature) - (the signal intensity at the relatively low detection temperature) * (the second reference value)]; or
   (iv) [(the signal intensity at the relatively high detection temperature) - (the signal intensity at the relatively low detection temperature) / (the second reference value)]; and

   determining the presence of the second target nucleic acid sequence by

   (i) [(the signal intensity at the relatively low detection temperature) - (the signal intensity at the relatively high detection temperature) * (the first reference value)];
   (ii) [(the signal intensity at the relatively low detection temperature) - (the signal intensity at the relatively high detection temperature) / (the first reference value)];
   (iii) [(the signal intensity at the relatively high detection temperature) - (the signal intensity at the relatively low detection temperature) * (the first reference value)]; or
   (iv) [(the signal intensity at the relatively high detection temperature) - (the signal intensity at the relatively low detection temperature) / (the first reference value)].

2. The method according to claim 1, wherein the step (b) is performed in a signal amplification process concomitantly with a nucleic acid amplification or without a nucleic acid amplification.

3. The method according to claim 1, wherein at least one of the two signal-generating means is a signal-generating means to generate a signal in a dependent manner on the formation of a duplex.

4. The method according to claim 1, wherein at least one of the two signal-generating means is a signal-generating means to generate a signal in a dependent manner on cleavage of a detection oligonucleotide.

5. The method according to claim 1, wherein the single reaction vessel further comprises at least one additional set each of which contains additional two signal-generating means for detection of target nucleic acid sequences other than the two target nucleic acid sequences; wherein the signals generated by each set of two signal-generating means in the vessel are differentiated from each other and the signals are detected by different types of detectors, respectively.

6. The method according to claim 1, wherein the two target nucleic acid sequences comprise a nucleotide variation and one of the two target nucleic acid sequences comprises one type of the nucleotide variation and the other comprises the other type of the nucleotide variation.

7. A method for SNP (single nucleotide polymorphism) genotyping of a nucleic acid sequence in a sample using different detection temperatures and reference values, comprising:

(a) providing (i) a first reference value for a homozygote composed of a first SNP allele which represents a ratio between in signals provided at a relatively high detection temperature and a relatively low detection temperature by a first signal-generating means; (ii) a second reference value for a homozygote composed of a second SNP allele which represents a ratio between signals provided at a relatively high detection temperature and a relatively low detection temperature by a second signal-generating means; and (iii) a third reference value for a heterozygote composed of the first SNP allele and the second SNP allele which represents a ratio between signals provided at a relatively high detection temperature and a relatively low detection temperature by the first signal-generating means and the second signal-generating means; wherein (i) a first reference value for a homozygote composed of a first SNP allele is obtained by (i-1) incubating the homozygote composed of the first SNP allele with a first signal-generating means for detection of the first SNP allele, (i-2) detecting signals at a relatively high detection temperature and a relatively low detection temperature, and (i-3) then obtaining a ratio between the signals detected at the relatively high detection temperature and the relatively low detection temperature, (ii) a second reference value for a homozygote composed of a second SNP allele is obtained by (ii-1) incubating the homozygote composed of the second SNP allele with a second signal-generating means for detection of the second SNP allele, (ii-2) detecting signals at the relatively high detection temperature and the relatively low detection temperature, and (ii-3) then obtaining a ratio between the signals detected at the relatively high detection temperature and the relatively low detection temperature; and (iii) a third reference value for a heterozygote composed of the first SNP allele and the second SNP allele is obtained by (iii-1) incubating the heterozygote composed of the first SNP allele and the second SNP allele with the first signal-generating means for detection of the first SNP allele and the second signal-generating means for detection of the second SNP allele, (iii-2) detecting signals at the relatively high detection temperature and the relatively low detection temperature, and (iii-3) then obtaining a ratio between the signals detected at the relatively high detection temperature and the relatively low detection temperature; wherein the first signal-generating means comprises an oligonucleotide hybridized with the first SNP allele nucleic acid sequence and the second signal-generating means comprises an oligonucleotide hybridized with the second SNP allele nucleic acid sequence; wherein the three reference values are different from each other;
(b) incubating the sample with the first signal-generating means and the second signal-generating means for the SNP alleles and detecting signals from the two signal-generating means at the relatively high detection temperature and the relatively low detection temperature; wherein the two signal-generating means generate signals at the relatively high detection temperature and at the relatively low detection temperature; wherein signals to be generated by the two signal-generating means are not differentiated by a single type of detector; and wherein the first signal-generating means comprises an oligonucleotide hybridized with the first SNP allele nucleic acid sequence and the second signal-generating means comprises an oligonucleotide hybridized with the second SNP allele nucleic acid sequence; wherein the relatively low detection temperature is a temperature at which the first signal-generating means does not generates signals indicating the presence of the second SNP allele and the second signal-generating means does not generates signals indicating the presence of the first SNP allele; wherein the incubating and detecting are performed by a real-time amplification reaction, not by melting analysis; and
(c) determining a SNP genotype by comparing a ratio between the signals detected at the relatively high detection temperature and the relatively low detection temperature in the step (b) with the reference values in the step (a).

8. A computer readable storage medium containing instructions for controlling a processor to determine the presence of at least one target nucleic acid sequence of two target nucleic acid sequences comprising a first target nucleic acid sequence and a second target nucleic acid sequence in a sample using different detection temperatures and reference values in the method according to any one of claims 1-6, the medium containing the instructions for the processor to:

(a) receive signals detected according to step (b) of any one of claims 1-6; and
(b) determine the presence of the first target nucleic acid sequence and the presence of the second target nucleic acid sequence according to step (c) of any one of claims 1-6;

wherein the first reference value and the second reference value are as defined in step (a) of any one of claims 1-6.

9. A computer readable storage medium containing instructions for controlling a processor to SNP (single nucleotide polymorphism) genotype a nucleic acid sequence in a sample using different detection temperatures and reference values in the method according to claim 7, the medium containing the instructions for the processor to:

(a) receive signals detected according to step (b) of claim 7; and
(b) determine a SNP genotype according to step (c) of claim 7; wherein the first reference value and the third reference value are as defined in step (a) of claim 7.

10. A device comprising (a) a computer processor and (b) the computer readable storage medium of claim 8 or 9 coupled to the computer processor.

**Patentansprüche**

1. Verfahren zum Nachweis mindestens einer Zielnukleinsäuresequenz von zwei Zielnukleinsäuresequenzen, die eine erste Zielnukleinsäuresequenz und eine zweite Zielnukleinsäuresequenz umfassen, in einer Probe unter Verwendung unterschiedlicher Nachweistemperaturen und Referenzwerte, umfassend:

(a) Bereitstellen (i) eines ersten Referenzwerts für die erste Zielnukleinsäuresequenz, der ein Verhältnis zwischen Signalen darstellt, die bei einer relativ hohen Nachweistemperatur und einer relativ niedrigen Nachweistemperatur durch ein erstes Signalerzeugungsmittel bereitgestellt werden, und (ii) eines zweiten Referenzwerts für die zweite Zielnukleinsäuresequenz, der ein Verhältnis zwischen Signalen darstellt, die bei der relativ hohen Nachweistemperatur und der relativ niedrigen Nachweistemperatur durch ein zweites Signalerzeugungsmittel bereitgestellt werden; wobei der erste Referenzwert von dem zweiten Referenzwert verschieden ist; wobei der erste Referenzwert erhalten wird durch (i) Inkubieren der ersten Zielnukleinsäuresequenz mit dem ersten Signalerzeugungsmittel zum Nachweis der ersten Zielnukleinsäuresequenz, (ii) Nachweisen von Signalen bei der relativ hohen Nachweistemperatur und der relativ niedrigen Nachweistemperatur und (iii) dann Erhalten eines Verhältnisses zwischen den bei der relativ hohen Nachweistemperatur und der relativ niedrigen Nachweistemperatur nachgewiesenen Signalen; wobei der zweite Referenzwert erhalten wird durch (i) Inkubieren der zweiten Zielnukleinsäuresequenz mit dem zweiten Signalerzeugungsmittel zum Nachweis der zweiten Zielnukleinsäuresequenz, (ii) Nachweisen von Signalen bei der relativ hohen Nachweistemperatur und der relativ niedrigen Nachweistemperatur und (iii) dann Erhalten eines Verhältnisses zwischen den bei der relativ hohen Nachweistemperatur und der relativ niedrigen Nachweistemperatur nachgewiesenen Signalen;
(b) Inkubieren der Probe mit dem ersten Signalerzeugungsmittel und dem zweiten Signalerzeugungsmittel zum Nachweis der zwei Zielnukleinsäuresequenzen und Nachweisen von Signalen von den zwei Signalerzeugungsmitteln bei der relativ hohen Nachweistemperatur und der relativ niedrigen Nachweistemperatur; wobei die zwei Signalerzeugungsmittel Signale bei der relativ hohen Nachweistemperatur und der relativ niedrigen Nachweistemperatur erzeugen; wobei Signale, die durch die zwei Signalerzeugungsmittel erzeugt werden sollen, nicht durch einen einzigen Detektortyp unterschieden werden; und wobei das erste Signalerzeugungsmittel ein Oligonukleotid umfasst, das mit der ersten Zielnukleinsäuresequenz hybridisiert ist, und das zweite Signalerzeugungsmittel ein Oligonukleotid umfasst, das mit der zweiten Zielnukleinsäuresequenz hybridisiert ist; wobei die relativ niedrige Nachweistemperatur eine Temperatur ist, bei der das erste Signalerzeugungsmittel keine Signale erzeugt, die das Vorhandensein der zweiten Zielnukleinsäuresequenz anzeigen, und das zweite Signalerzeugungsmittel keine Signale erzeugt, die das Vorhandensein der ersten Zielnukleinsäuresequenz anzeigen; wobei das Inkubieren und Nachweisen durch eine Echtzeit-Amplifikationsreaktion, nicht durch Schmelzanalyse, durchgeführt werden; und

(c) Bestimmen des Vorhandenseins der ersten Zielnukleinsäuresequenz durch

(i) [(die Signalintensität bei der relativ niedrigen Nachweistemperatur) - (die Signalintensität bei der relativ hohen Nachweistemperatur) * (der zweite Referenzwert)];
(ii) [(die Signalintensität bei der relativ niedrigen Nachweistemperatur) - (die Signalintensität bei der relativ hohen Nachweistemperatur) / (der zweite Referenzwert)];
(iii) [(die Signalintensität bei der relativ hohen Nachweistemperatur) - (die Signalintensität bei der relativ niedrigen Nachweistemperatur) * (der zweite Referenzwert)]; oder
(iv) [(die Signalintensität bei der relativ hohen Nachweistemperatur) - (die Signalintensität bei der relativ niedrigen Nachweistemperatur) / (der zweite Referenzwert)]; und

Bestimmen des Vorhandenseins der zweiten Zielnukleinsäuresequenz durch

(i) [(die Signalintensität bei der relativ niedrigen Nachweistemperatur) - (die Signalintensität bei der relativ hohen Nachweistemperatur) * (der erste Referenzwert)];
(ii) [(die Signalintensität bei der relativ niedrigen Nachweistemperatur) - (die Signalintensität bei der relativ hohen Nachweistemperatur) / (der erste Referenzwert)];
(iii) [(die Signalintensität bei der relativ hohen Nachweistemperatur) - (die Signalintensität bei der relativ niedrigen Nachweistemperatur) * (der erste Referenzwert)]; oder
(iv) [(die Signalintensität bei der relativ hohen Nachweistemperatur) - (die Signalintensität bei der relativ niedrigen Nachweistemperatur) / (der erste Referenzwert)].

2. Verfahren nach Anspruch 1, wobei der Schritt (b) in einem Signalamplifikationsprozess gleichzeitig mit einer Nukleinsäureamplifikation oder ohne eine Nukleinsäureamplifikation durchgeführt wird.

3. Verfahren nach Anspruch 1, wobei mindestens eines der beiden Signalerzeugungsmittel ein Signalerzeugungsmittel ist, um ein Signal in Abhängigkeit von der Bildung eines Duplexs zu erzeugen.

4. Verfahren nach Anspruch 1, wobei mindestens eines der beiden Signalerzeugungsmittel ein Signalerzeugungsmittel ist, um ein Signal in Abhängigkeit von der Spaltung eines Detektionsoligonukleotids zu erzeugen.

5. Verfahren nach Anspruch 1, wobei das einzelne Reaktionsgefäß ferner mindestens einen zusätzlichen Satz umfasst, von denen jeder zusätzliche zwei Signalerzeugungsmittel zum Nachweis von anderen Zielnukleinsäuresequenzen als den zwei Zielnukleinsäuresequenzen enthält; wobei die Signale, die durch jeden Satz von zwei Signalerzeugungsmitteln in dem Gefäß erzeugt werden, voneinander unterschieden werden und die Signale jeweils durch unterschiedliche Detektortypen nachgewiesen werden.

6. Verfahren nach Anspruch 1, wobei die zwei Zielnukleinsäuresequenzen eine Nukleotidvariation umfassen und eine der zwei Zielnukleinsäuresequenzen einen Typ der Nukleotidvariation umfasst und die andere den anderen Typ der Nukleotidvariation umfasst.

7. Verfahren zur SNP (Einzelnukleotidpolymorphismus)-Genotypisierung einer Nukleinsäuresequenz in einer Probe unter Verwendung unterschiedlicher Nachweistemperaturen und Referenzwerte, umfassend:

(a) Bereitstellen (i) eines ersten Referenzwerts für einen Homozygoten, der aus einem ersten SNP-Allel besteht, das ein Verhältnis zwischen Signalen darstellt, die bei einer relativ hohen Nachweistemperatur und einer relativ niedrigen Nachweistemperatur durch ein erstes Signalerzeugungsmittel bereitgestellt werden; (ii) eines zweiten Referenzwerts für einen Homozygoten, der aus einem zweiten SNP-Allel besteht, das ein Verhältnis zwischen Signalen darstellt, die bei einer relativ hohen Nachweistemperatur und einer relativ niedrigen Nachweistemperatur durch ein zweites Signalerzeugungsmittel bereitgestellt werden; und (iii) eines dritten Referenzwerts für einen Heterozygoten, der aus dem ersten SNP-Allel und dem zweiten SNP-Allel besteht, das ein Verhältnis zwischen Signalen darstellt, die bei einer relativ hohen Nachweistemperatur und einer relativ niedrigen Nachweistemperatur durch das erste Signalerzeugungsmittel und das zweite Signalerzeugungsmittel bereitgestellt werden; wobei (i) ein erster Referenzwert für einen Homozygoten, der aus einem ersten SNP-Allel besteht, erhalten wird durch (i-1) Inkubieren des Homozygoten, der aus dem ersten SNP-Allel besteht, mit einem ersten Signalerzeugungsmittel zum Nachweis des ersten SNP-Allels, (i-2) Nachweisen von Signalen bei einer relativ hohen Nachweistemperatur und einer relativ niedrigen Nachweistemperatur und (i-3) anschließendes Erhalten eines Verhältnisses zwischen den bei der relativ hohen Nachweistemperatur und der relativ niedrigen Nachweistemperatur nachgewiesenen Signalen, (ii) ein zweiter Referenzwert für einen Homozygoten, der aus einem zweiten SNP-Allel besteht, erhalten wird durch (ii-1) Inkubieren des Homozygoten, der aus dem zweiten SNP-Allel besteht, mit einem zweiten Signalerzeugungsmittel zum Nachweis des zweiten SNP-Allels, (ii-2) Nachweisen von Signalen bei der relativ hohen Nachweistemperatur und der relativ niedrigen Nachweistemperatur und (ii-3) anschließendes Erhalten eines Verhältnisses zwischen den bei der relativ hohen Nachweistemperatur und der relativ niedrigen Nachweistemperatur nachgewiesenen Signalen; und (iii) ein dritter Referenzwert für einen Heterozygoten, der aus dem ersten SNP-Allel und dem zweiten SNP-Allel besteht, erhalten wird durch (iii-1) Inkubieren des Heterozygoten, der aus dem ersten SNP-Allel und dem zweiten SNP-Allel besteht, mit dem ersten Signalerzeugungsmittel zum Nachweis des ersten SNP-Allels und dem zweiten Signalerzeugungsmittel zum Nachweis des zweiten SNP-Allels, (iii-2) Nachweisen von Signalen bei der relativ hohen Nachweistemperatur und der relativ niedrigen Nachweistemperatur und (iii-3) anschließendes Erhalten eines Verhältnisses zwischen den bei der relativ hohen Nachweistemperatur und der relativ niedrigen Nachweistemperatur nachgewiesenen Signalen; wobei das erste Signalerzeugungsmittel ein Oligonukleotid umfasst, das mit der ersten SNP-Allel-Nukleinsäuresequenz hybridisiert ist, und das zweite Signalerzeugungsmittel ein Oligonukleotid umfasst, das mit der zweiten SNP-Allel-Nukleinsäuresequenz hybridisiert ist; wobei die drei Referenzwerte voneinander verschieden sind;

(b) Inkubieren der Probe mit dem ersten Signalerzeugungsmittel und dem zweiten Signalerzeugungsmittel für die SNP-Allele und Nachweisen von Signalen von den zwei Signalerzeugungsmitteln bei der relativ hohen Nachweistemperatur und der relativ niedrigen Nachweistemperatur; wobei die zwei Signalerzeugungsmittel Signale bei der relativ hohen Nachweistemperatur und der relativ niedrigen Nachweistemperatur erzeugen; wobei Signale, die durch die zwei Signalerzeugungsmittel erzeugt werden sollen, nicht durch einen einzigen Detektortyp unterschieden werden; und wobei das erste Signalerzeugungsmittel ein Oligonukleotid umfasst, das mit der ersten SNP-Allel-Nukleinsäuresequenz hybridisiert ist, und das zweite Signalerzeugungsmittel ein Oligonukleotid umfasst, das mit der zweiten SNP-Allel-Nukleinsäuresequenz hybridisiert ist; wobei die relativ niedrige Nachweistemperatur eine Temperatur ist, bei der das erste Signalerzeugungsmittel keine Signale erzeugt, die das Vorhandensein des zweiten SNP-Allels anzeigen, und das zweite Signalerzeugungsmittel keine Signale erzeugt, die das Vorhandensein des ersten SNP-Allels anzeigen; wobei das Inkubieren und Nachweisen durch eine Echtzeit-Amplifikationsreaktion, nicht durch Schmelzanalyse, durchgeführt werden; und

(c) Bestimmen eines SNP-Genotyps durch Vergleichen eines Verhältnisses zwischen den bei der relativ hohen Nachweistemperatur und der relativ niedrigen Nachweistemperatur in Schritt (b) nachgewiesenen Signalen mit den Referenzwerten in Schritt (a).

8. Computerlesbares Speichermedium, das Anweisungen zum Steuern eines Prozessors enthält, um das Vorhandensein mindestens einer Zielnukleinsäuresequenz von zwei Zielnukleinsäuresequenzen, die eine erste Zielnukleinsäuresequenz und eine zweite Zielnukleinsäuresequenz umfassen, in einer Probe unter Verwendung unterschiedlicher Nachweistemperaturen und Referenzwerte in dem Verfahren nach einem der Ansprüche 1-6 zu bestimmen, wobei das Medium die Anweisungen für den Prozessor enthält zum:

(a) Empfangen von Signalen, die gemäß Schritt (b) nach einem der Ansprüche 1-6 nachgewiesen wurden; und
(b) Bestimmen des Vorhandenseins der ersten Zielnukleinsäuresequenz und des Vorhandenseins der zweiten Zielnukleinsäuresequenz gemäß Schritt (c) nach einem der Ansprüche 1-6;

wobei der erste Referenzwert und der zweite Referenzwert wie in Schritt (a) nach einem der Ansprüche 1-6 definiert sind.

9. Computerlesbares Speichermedium, das Anweisungen zum Steuern eines Prozessors enthält, um eine Nukleinsäuresequenz in einer Probe unter Verwendung unterschiedlicher Nachweistemperaturen und Referenzwerte in dem Verfahren nach Anspruch 7 durch SNP (Einzelnukleotidpolymorphismus) zu genotypisieren, wobei das Medium die Anweisungen für den Prozessor enthält zum:

(a) Empfangen von Signalen, die gemäß Schritt (b) nach Anspruch 7 nachgewiesen wurden; und
(b) Bestimmen eines SNP-Genotyps gemäß Schritt (c) nach Anspruch 7; wobei der erste Referenzwert und der dritte Referenzwert wie in Schritt (a) nach Anspruch 7 definiert sind.

10. Vorrichtung, umfassend (a) einen Computerprozessor und (b) das computerlesbare Speichermedium nach Anspruch 8 oder 9, das mit dem Computerprozessor gekoppelt ist.

**Revendications**

1. Procédé de détection, dans un échantillon, d'au moins une séquence d'acide nucléique cible parmi deux séquences d'acide nucléique cibles comprenant une première séquence d'acide nucléique cible et une deuxième séquence d'acide nucléique cible, le procédé étant effectué en utilisant différentes températures de détection et valeurs de référence et comprenant les étapes suivantes :

(a) obtention, (i) pour la première séquence d'acide nucléique cible, d'une première valeur de référence qui représente un rapport entre les signaux respectivement produits avec un premier moyen générant un signal à une température de détection relativement élevée et à une température de détection relativement basse et (ii) pour la deuxième séquence d'acide nucléique cible, d'une deuxième valeur de référence qui représente un rapport entre les signaux respectivement produits avec un deuxième moyen générant un signal à une température de détection relativement élevée et à une température de détection relativement basse ; où la première valeur de référence est différente de la deuxième valeur de référence ; où la première valeur de référence est obtenue par (i) incubation de la première séquence d'acide nucléique cible avec le premier moyen générant un signal pour détecter la première séquence d'acide nucléique cible, (ii) détection des signaux produits à la température de détection relativement élevée et à la température de détection relativement basse et (iii) calcul d'un rapport entre les signaux produits à la température de détection relativement élevée et à la température de détection relativement basse ; où la deuxième valeur de référence est obtenue par (i) incubation de la deuxième séquence d'acide nucléique cible avec le deuxième moyen générant un signal pour détecter la deuxième séquence d'acide nucléique cible, (ii) détection des signaux produits à la température de détection relativement élevée et à la température de détection relativement basse et (iii) calcul d'un rapport entre les signaux produits à la température de détection relativement élevée et à la température de détection relativement basse ;

(b) incubation de l'échantillon avec le premier moyen générant un signal et le deuxième moyen générant un signal pour détecter les deux séquences d'acide nucléique cibles et pour détecter les signaux respectivement produits par les deux moyens générant un signal à la température de détection relativement élevée et à la température de détection relativement basse ; où les deux moyens générant un signal génèrent des signaux à la température de détection relativement élevée et à la température de détection relativement basse ; où les signaux générés par les deux moyens générant un signal ne sont pas différenciés par un type de détecteur unique ; et où le premier moyen générant un signal comprend un oligonucléotide hybridé à la première séquence d'acide nucléique cible et le deuxième moyen générant un signal comprend un oligonucléotide hybridé à la deuxième séquence d'acide nucléique cible ; où la température de détection relativement basse est une température à laquelle le premier moyen générant un signal ne génère pas de signaux indicateurs de la présence de la deuxième séquence d'acide nucléique cible et à laquelle le deuxième moyen générant un signal ne génère pas de signaux indicateurs de la présence de la première séquence d'acide nucléique cible ; où les étapes d'incubation et de détection sont effectuées par une réaction d'amplification en temps réel et non par une analyse des courbes de fusion ; et

(c) détermination, comme suit, de la présence de la première séquence d'acide nucléique cible (i) [(intensité du signal à la température de détection relativement basse) - (intensité du signal à la température de détection relativement élevée) * (deuxième valeur de référence)] ;

(ii) [(intensité du signal à la température de détection relativement basse) - (intensité du signal à la température de détection relativement élevée) / (deuxième valeur de référence)] ;
(iii) [(intensité du signal à la température de détection relativement élevée) - (intensité du signal à la température de détection relativement basse) * (deuxième valeur de référence)] ; ou
(iv) [(intensité du signal à la température de détection relativement élevée) - (intensité du signal à la température de détection relativement basse) / (deuxième valeur de référence)] ; et

détermination, comme suit, de la présence de la deuxième séquence d'acide nucléique cible

(i) [(intensité du signal à la température de détection relativement basse) - (intensité du signal à la température de détection relativement élevée) * (première valeur de référence)] ;
(ii) [(intensité du signal à la température de détection relativement basse) - (intensité du signal à la température de détection relativement élevée) / (première valeur de référence)] ;
(iii) [(intensité du signal à la température de détection relativement élevée) - (intensité du signal à la température de détection relativement basse) * (première valeur de référence)] ; ou
(iv) [(intensité du signal à la température de détection relativement élevée) - (intensité du signal à la température de détection relativement basse) / (première valeur de référence)].

**2.** Procédé selon la revendication 1, où l'étape (b) est effectuée lors d'un processus d'amplification du signal en même temps que l'amplification d'un acide nucléique ou sans amplification d'un acide nucléique.

**3.** Procédé selon la revendication 1, où au moins un des deux moyens générant un signal est un moyen générant un signal conçu pour générer un signal d'une manière qui dépend de la formation d'un duplex.

**4.** Procédé selon la revendication 1, où au moins un des deux moyens générant un signal est un moyen générant un signal conçu pour générer un signal d'une manière qui dépend du clivage d'un oligonucléotide de détection.

**5.** Procédé selon la revendication 1, où la chambre de réaction unique comprend en outre au moins une série supplémentaire contenant chacune deux autres moyens générant un signal permettant de détecter des séquences d'acide nucléique cibles autres que les deux séquences d'acide nucléique cibles ; où les signaux générés par chaque série de deux moyens générant un signal sont différenciés les uns des autres et où les signaux sont détectés par différents types de détecteurs, respectivement.

**6.** Procédé selon la revendication 1, où les deux séquences d'acide nucléique cibles comprennent une variation nucléotidique et où une des deux séquences d'acide nucléique cibles comprend un type de variation nucléotidique et l'autre comprend l'autre type de variation nucléotidique.

**7.** Procédé d'identification d'un SNP (pour *single nucleotide polymorphism,* polymorphisme mononucléotidique) par génotypage d'une séquence d'acide nucléique dans un échantillon en utilisant différentes températures de détection et valeurs de référence, le procédé comprenant les étapes suivantes :

(a) obtention (i), pour un homozygote composé d'un allèle d'un premier SNP, d'une première valeur de référence qui représente un rapport entre les signaux respectivement produits à une température de détection relativement élevée et à une température de détection relativement basse avec un premier moyen générant un signal ; (ii), pour un homozygote composé d'un allèle d'un deuxième SNP, d'une deuxième valeur de référence qui représente un rapport entre les signaux respectivement produits à une température de détection relativement élevée et à une température de détection relativement basse avec un deuxième moyen générant un signal ; et (iii), pour un hétérozygote composé d'un allèle du premier SNP et d'un allèle du deuxième SNP, d'une troisième valeur de référence qui représente un rapport entre les signaux respectivement produits à une température de détection relativement élevée et à une température de détection relativement basse par le premier moyen générant un signal et le deuxième moyen générant un signal ; où (i) une première valeur de référence pour un homozygote composé d'un allèle d'un premier SNP est obtenue par (i-1) incubation de l'homozygote composé d'un allèle du premier SNP avec un premier moyen générant un signal pour détecter l'allèle du premier SNP, (i-2) détection des signaux produits à une température de détection relativement élevée et à une température de détection relativement basse puis (i-3) calcul d'un rapport entre les signaux détectés à la température de détection relativement élevée et à la température de détection relativement basse, (ii) une deuxième valeur de référence pour un homozygote composé d'un allèle d'un deuxième SNP est obtenue par (ii-1) incubation de l'homozygote composé d'un allèle d'un deuxième SNP avec un deuxième moyen générant un signal pour détecter l'allèle du deuxième SNP, (ii-2) détection des signaux produits à une température de détection relativement élevée et à une température de détection relativement basse puis (ii-3) calcul d'un rapport entre les signaux détectés à la température de détection relativement élevée et à la température de détection relativement basse ; et (iii) une troisième valeur de référence pour un hétérozygote composé d'un allèle du premier SNP et d'un allèle du deuxième SNP est obtenue par (iii-1) incubation de l'hétérozygote composé d'un allèle du premier SNP et d'un allèle du deuxième SNP avec le premier moyen générant un signal pour détecter l'allèle du premier SNP et avec le deuxième moyen générant un signal pour détecter l'allèle du deuxième SNP, (iii-2) détection des signaux produits à une température de détection relativement élevée et à une température de détection relativement basse puis (iii-3) calcul d'un rapport entre les signaux détectés à la température de détection relativement élevée et à la température de détection relativement basse ; où le premier moyen générant un signal comprend un oligonucléotide hybridé à la séquence d'acide nucléique de l'allèle du premier SNP et où le deuxième moyen générant un signal comprend un oligonucléotide hybridé à la séquence d'acide nucléique de l'allèle du deuxième SNP ; où les trois valeurs de référence sont différentes les unes des autres ;

(b) incubation de l'échantillon avec le premier moyen générant un signal et le deuxième moyen générant un signal pour les allèles des SNP et détection des signaux produits par les deux moyens générant un signal à la température de détection relativement élevée et à la température de détection relativement basse ; où les deux moyens générant un signal génèrent des signaux à la température de détection relativement élevée et à la température de détection relativement basse ; où les signaux générés par les deux moyens générant un signal ne sont pas différenciés par un type de détecteur unique ; et où le premier moyen générant un signal comprend un oligonucléotide hybridé à la séquence d'acide nucléique de l'allèle du premier SNP et le deuxième moyen générant un signal comprend un oligonucléotide hybridé à la séquence d'acide nucléique de l'allèle du deuxième SNP ; où la température de détection relativement basse est une température à laquelle le premier moyen générant un signal ne génère pas de signaux indicateurs de la présence de l'allèle du deuxième SNP et à laquelle le deuxième moyen générant un signal ne génère pas de signaux indicateurs de la présence de l'allèle du premier SNP ; où les étapes d'incubation et de détection sont effectuées par une réaction d'amplification en temps réel et non par une analyse des courbes de fusion ; et

(c) détermination du génotype d'un SNP par comparaison d'un rapport entre les signaux détectés à l'étape (b) à la température de détection relativement élevée et à la température de détection relativement basse aux valeurs de référence obtenues à l'étape (a).

8. Support de stockage lisible par ordinateur contenant des instructions permettant de contrôler un processeur pour déterminer la présence, dans un échantillon, d'au moins une séquence d'acide nucléique cible parmi deux séquences d'acide nucléique cibles comprenant une première séquence d'acide nucléique cible et une deuxième séquence d'acide nucléique cible en utilisant différentes températures et valeurs de référence conformément au procédé selon l'une quelconque des revendications 1-6, le support contenant les instructions qui conduisent le processeur à :

(a) recevoir les signaux détectés conformément à l'étape (b) de l'une quelconque des revendications 1-6 ; et
(b) déterminer la présence de la première séquence d'acide nucléique cible et de la deuxième séquence d'acide nucléique cible conformément à l'étape (c) de l'une quelconque des revendications 1-6 ;

où la première valeur de référence et la deuxième valeur de référence sont telles que définies à l'étape (a) de l'une quelconque des revendications 1-6 .

9. Support de stockage lisible par ordinateur contenant des instructions permettant de contrôler un processeur pour déterminer le génotype d'un SNP (polymorphisme mononucléotidique) dans une séquence d'acide nucléique dans un échantillon en utilisant différentes températures et valeurs de référence conformément au procédé selon la revendication 7, le support contenant les instructions qui conduisent le processeur à :

(a) recevoir les signaux détectés conformément à l'étape (b) de la revendication 7 ; et
(b) déterminer le génotype d'un SNP conformément à l'étape (b) de la revendication 7 ; où la première valeur de référence et la troisième valeur de référence sont telles que définies à l'étape (a) de la revendication 7.

10. Dispositif comprenant (a) un processeur d'ordinateur et (b) le support de stockage lisible par ordinateur de la revendication 8 ou 9 connecté au processeur d'ordinateur.

# Fig. 1A

**Detection temperature**

1)   Template is genomic DNA of *Neisseria gonorrhoeae* (NG) and *Chlamydia trachomatis* (CT). NTC is No Template Control

# Fig. 1B

| Template[1] | End-RFU[2] | | Reference value[3] |
|---|---|---|---|
| | 72°C | 60°C | |
| NG | 547 | 997 | 1.8 |
| CT | 375 | 2177 | 5.8 |

[1] Template is genomic DNA of *Neisseria gonorrhoeae* (NG) and *Chlamydia trachomatis* (CT).

[2] End-RFU represents relative fluorescence units at 50th cycle.

[3] Reference value represents the ratio of End-RFU at 60°C to End-RFU at 72°C.

# Fig. 1C

## Determining the presence of NG

### (i) 72°C − (60°C ÷ RV[2]) of CT)     (ii) 60°C − (72°C X RV[2]) of CT)

Template[1]

[1] Template is genomic DNA of *Neisseria gonorrhoeae* (NG) and *Chlamydia trachomatis* (CT). NTC is No Template Control

[2] RV means reference value.

# Fig. 1D

Determining the presence of CT

(i) 72°C – (60°C ÷ RV[2] of NG)  (ii) 60°C – (72°C X RV[2] of NG)

Template[1]

[1] Template is genomic DNA of *Neisseria gonorrhoeae* (NG) and *Chlamydia trachomatis* (CT). NTC is No Template Control

[2] RV means reference value.

# Fig. 2A

## Detection temperature

<sup>1)</sup> Template is genomic DNA of *Neisseria gonorrhoeae* (NG) and *Chlamydia trachomatis* (CT). NTC is No Template Control

# Fig. 2B

| Template[1] | End-RFU[2] | | Reference value[3] |
|:---:|:---:|:---:|:---:|
| | 67.8°C | 60°C | |
| NG | 364 | 2214 | 6.1 |
| CT | 1907 | 1986 | 1.0 |

[1] Template is genomic DNA of *Neisseria gonorrhoeae* (NG) and *Chlamydia trachomatis* (CT).

[2] End-RFU represents relative fluorescence units at 50th cycle.

[3] Reference value represents the ratio of End-RFU at 60°C to End-RFU at 67.8°C.

# Fig. 2C

## Determining the presence of NG

**(i) 67.8°C – (60°C ÷ RV²⁾ of CT)**  **(ii) 60°C – (67.8°C X RV²⁾ of CT)**

Template¹⁾

NG

CT

CT + NG

NTC

¹⁾ Template is genomic DNA of *Neisseria gonorrhoeae* (NG) and *Chlamydia trachomatis* (CT). NTC is No Template Control

²⁾ RV means reference value.

# Fig. 2D

## Determining the presence of CT

(i) 67.8°C – (60°C ÷ RV[2] of NG)      (ii) 60°C – (67.8°C X RV[2] of NG)

Template[1]

[1] Template is genomic DNA of *Neisseria gonorrhoeae* (NG) and *Chlamydia trachomatis* (CT). NTC is No Template Control

[2] RV means reference value.

# Fig. 3A

**Detection temperature**

1)  Target is human genomic DNA of wild, mutant, and hetero-type MTHFR (C677T).
NTC is No Template Control.

# Fig. 3B

| Template[1] | End-RFU[2] | | Reference value[3] |
|---|---|---|---|
| | 64°C | 60°C | |
| Wild | 1466 | 1454 | 1.0 |
| Hetero | 873 | 1142 | 1.3 |
| Mutant | 240 | 655 | 2.7 |

[1] Target is human genomic DNA of wild, mutant, and hetero-type MTHFR (C677T).
[2] End-RFU represents relative fluorescence units at 50th cycle.
[3] Reference value represents the ratio of End-RFU at 60°C to End-RFU at 64°C.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7537886 B
- US 5210015 A
- US 5538848 A
- US 6117635 A
- US 6326145 B
- WO 2011078441 A
- US 5691142 A
- US 6358691 B
- US 6194149 B
- WO 2012096523 A
- WO 2013115442 A
- KR 2013012312 W
- WO 2015147370 A1
- US 6977295 B
- WO 2012134195 A
- US 7309573 B
- US 7348141 B
- US 3996345 A
- US 4351760 A
- US 5432272 A
- US 5965364 A
- US 6001983 A
- US 6037120 A
- US 7422850 B
- US 4683195 A, Mullis
- US 4683202 A
- US 4800159 A
- WO 9001069 A
- EP 439182 A
- WO 9300447 A
- US 5556751 A
- EP 497272 A
- US 5888779 A
- WO 2011037306 A

**Non-patent literature cited in the description**

- **TYAGI et al.** *Nature Biotechnology*, March 1996, vol. 14
- **FRENCH DJ et al.** *Mol. Cell Probes*, 2001, vol. 15 (6), 363-374
- **BERNAD et al.** *Clin Chem*, 2000, vol. 46, 147-148
- **NAZARENKO et al.** *Nucleic Acids Research*, 1997, vol. 25 (12), 2516-2521
- **WHITCOMBE et al.** *Nature Biotechnology*, August 1999, vol. 17, 804-807
- **WHITCOMBE et al.** *Nature Biotechnology*, 1999, vol. 17, 804-807
- **SHERRILL C B et al.** *Journal of the American Chemical Society*, 2004, vol. 126, 4550-45569
- **BERNARD, P.S. et al.** *Anal. Biochem.*, 1999, vol. 273, 221
- **SALVATORE et al.** *Nucleic Acids Research*, 2002, vol. 30 (21), e122
- **JOHANSSON et al.** *J. AM. CHEM. SOC*, 2002, vol. 124, 6950-6956
- **PESCE et al.** Fluorescence Spectroscopy. Marcel Dekker, 1971
- **WHITE et al.** Fluorescence Analysis: A Practical Approach. Marcel Dekker, 1970
- **BERLMAN**. Handbook of Fluorescence Spectra of Aromatic Molecules. Academic Press, 1971
- **GRIFFITHS**. Color AND Constitution of Organic Molecules. Academic Press, 1976
- Indicators. Pergamon Press, 1972
- Molecular Probes. **HAUGLAND** ; **EUGENE**. Handbook of Fluorescent Probes and Research Chemicals. 1992
- **PRINGSHEIM**. Fluorescence and Phosphorescence. Interscience Publishers, 1949
- Molecular Probes. **HAUGLAND, R. P.** ; **EUGENE**. Handbook of Fluorescent Probes and Research Chemicals. 1996
- **SHERRILL C B et al.** Plexor method. *Journal of the American Chemical Society*, 2004, vol. 126, 4550-45569
- **JOSEPH SAMBROOK et al.** Molecular Cloning, A Laboratory Manual. Cold Spring Harbor Laboratory Press, Cold Spring Harbor, 2001
- **NOONAN, K. F et al.** *Nucleic Acids Res.*, 1988, vol. 16, 10366
- **SAIKI et al.** *Science*, 1985, vol. 230, 1350-1354
- **WIEDMANN M et al.** Ligase chain reaction (LCR)-overview and applications. *PCR Methods and Applications*, February 1994, vol. 3 (4), 51-64
- **CAHILL P et al.** *Clin Chem.*, 1991, vol. 37 (9), 1482-5
- **G T WALKER et al.** *Nucleic Acids Res.*, 1992, vol. 20 (7), 16911696
- **COMPTON, J.** *Nature*, 1991, vol. 350 (6313), 912

- **HOFMANN WP et al.** *J Clin Virol.*, 2005, vol. 32 (4), 289-93
- **HUTCHISON C.A. et al.** Rolling Circle Amplification. *Proc. Natl Acad. Sci. USA.*, 2005, vol. 102, 1733217336
- **DUCK P et al.** *Biotechniques*, 1990, vol. 9, 142-148